(19) Europäisches Patentamt / European Patent Office / Office européen des brevets

(11) **EP 1 660 085 B1**

(12) **EUROPEAN PATENT SPECIFICATION**

(45) Date of publication and mention of the grant of the patent:
**15.10.2008 Bulletin 2008/42**

(21) Application number: **04763848.1**

(22) Date of filing: **06.08.2004**

(51) Int Cl.:
***A61K 31/444*** *(2006.01)* ***A61P 35/00*** *(2006.01)*
***A61P 25/28*** *(2006.01)* ***A61P 31/12*** *(2006.01)*
***A61P 37/00*** *(2006.01)* ***A61P 13/08*** *(2006.01)*
***A61P 17/06*** *(2006.01)* ***A61P 9/10*** *(2006.01)*
***A61P 11/00*** *(2006.01)* ***A61P 19/02*** *(2006.01)*
***A61P 13/12*** *(2006.01)* ***A61K 45/06*** *(2006.01)*
***C07D 519/00*** *(2006.01)* ***C07D 471/04*** *(2006.01)*

(86) International application number:
**PCT/EP2004/008811**

(87) International publication number:
**WO 2005/013986 (17.02.2005 Gazette 2005/07)**

(54) **PYRIDYLPYRROLE DERIVATIVES ACTIVE AS KINASE INHIBITORS**

PYRIDYLPYRROL-DERIVATE ALS WIRKSAME KINASE-HEMMER

DERIVES DE PYRIDYLPYRROLE COMME INHIBITEURS DE KINASE

(84) Designated Contracting States:
**AT BE BG CH CY CZ DE DK EE ES FI FR GB GR HU IE IT LI LU MC NL PL PT RO SE SI SK TR**

(30) Priority: **08.08.2003 US 494076 P**
**18.06.2004 US 580836 P**

(43) Date of publication of application:
**31.05.2006 Bulletin 2006/22**

(73) Proprietor: **Pfizer Italia S.r.l.**
**04010 Latina (IT)**

(72) Inventors:
- **VANOTTI, Ermes**
  **I-20148 Milano (IT)**
- **D'ALESSIO, Roberto**
  **I-20092 Cinisello Balsamo (IT)**
- **TIBOLLA, Marcellino**
  **I-20030 Senago (IT)**
- **VARASI, Mario**
  **I-20142 Milan (IT)**
- **MONTAGNOLI, Alessia**
  **I-20151 Milan (IT)**
- **SANTOCANALE, Corrado**
  **I-22070 Appiano Gentile (IT)**
- **MARTINA, Katia**
  **I-10066 Torre Pellice (IT)**
- **MENICHINCHERI, Maria**
  **I-20124 Milan (IT)**

(74) Representative: **Modiano, Micaela Nadia et al**
**Modiano Josif Pisanty & Staub Ltd**
**Thierschstrasse 11**
**80538 München (DE)**

(56) References cited:
**WO-A-01/62251** **WO-A-02/12242**
**WO-A-03/027114** **WO-A-20/04058762**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 660 085 B1

## Description

## BACKGROUND OF THE INVENTION

### Field of the Invention

The present invention relates to pyridylpyrrole derivatives, to pharmaceutical compositions comprising them and to their use as therapeutic agents, particularly In the treatment of cancer and cell proliferation disorders.

### Discussion of the Background

The malfunctioning of protein kinases (PKs) is the hallmark of numerous diseases. A large share of the oncogenes and proto-oncogenes Involved in human cancers code for PKs. The enhanced activities of PKs are also Implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis.

PKs are also implicated in inflammatory conditions and in the multiplication of viruses and parasites. PKs may also play a major role in the pathogenesis and development of neurodegenerative disorders.

For a general reference to PKs malfunctioning or disregulation see, for instance, Current Opinion in Chemical Biology 1999, 3, 459 - 465.

Among the several protein kinases known in the art as being implicated in the growth of cancer cells is Cdc7, an evolutionary conserved serine-threonine kinase which plays a pivotal role in linking cell cycle regulation to genome duplication, being essential for the firing of DNA replication origins (see Montagnoll A. at al., EMBO Journal, Vol. 21, No. 12, pp. 3171-3181. 2002).

WO 03/027114 A discloses 1,5,6,7-tetrahydro-4H-pyrrolo [3,2-c]pyridin-4-ones which can be substituted with a pyridine ring in position 2. The compounds can be used for the treatment of obesity and obesity-related disorders, including atherosclerosis, osteoathritis and cancer.

## SUMMARY OF THE INVENTION

It is an object of the invention to provide compounds which are useful, in therapy, as agents against a host of diseases caused by and/or associated to a disregulated protein kinase activity and, more particularly, Cdk2 and Cdc7 activity.

It is another object to provide compounds, which are endowed with protein kinase inhibiting activity and, more particularly, Cdk2 and Cdc7 inhibiting activity.

The present inventors have now discovered that some pyridylpyrroles, and derivatives thereof, are endowed with protein kinase inhibiting activity, e.g. Cdk2 and especially Cdc7 inhibiting activity.

More specifically, the compounds of this invention are useful in the treatment of a variety of cancers including: carcinoma such as bladder, breast, colon, kidney, liver, lung, including small cell lung cancer, esophagus, gall-bladder, ovary, pancreas, stomach, cervix, thyroid, prostate, and skin, including squamous cell carcinoma; hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.

Due to the key role of PKs, Cdk2 and Cdc7 in the regulation of cellular proliferation, these pyridylpyrroles are also useful in the treatment of a variety of cell proliferative disorders such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis.

The compounds of the invention can be also active as inhibitors of other protein kinases such as, for instance, protein kinase C in different isoforms, Met, PAK-4, PAK-5, ZC-1, STLK-2, DDR-2, Aurora 1, Aurora 2, Bub-1, PLK, Chk1, Chk2, HER2, raf1, MEK1, MAPK, EGF-R, PDGF-R, FGF-R, IGF-R, VEGF-R, PI3K, weel kinase, Src, Abl, Akt, ILK, MK-2, IKK-2, Cdk in different isoforms, Nek, and thus be effective in the treatment of diseases associated with other protein kinases.

Accordingly, in a first embodiment, the present invention provides the use of a compound of formula (I) for the manufacture of a medicament for treating cell proliferative disorders caused by and/or associated with an altered protein

kinase activity as defined in claim 1, wherein the compound is to be administered in an effective amount to a mammal in need thereof,

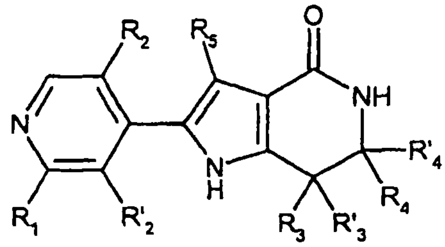

(I)

wherein
$R_1$ is a hydrogen atom, amino, $C_3$-$C_7$ cycloalkylamino;
$R_2$ and $R'_2$ are, each independently, a hydrogen or halogen atom or a straight or branched $C_1$-$C_6$ alkyl group; or, taken together with the pyridine bond to which they are linked, $R_1$ and $R'_2$ may form a divalent -NH-CH=CH- group;
$R_3$, $R'_3$, $R_4$ and $R'_4$ are, each independently, a hydrogen atom or a group selected from straight or branched $C_1$-$C_6$ alkyl, $C_3$-$C_6$ cycloalkyl, heterocyclyl, aryl, cycloalkyl-alkyl, heterocyclyl-alkyl or aryl-alkyl; or $R_3$ and $R_3$'or $R_4$ and $R_4$', taken together, form a $C_3$-$C_6$ cyclic alkyl group;
$R_5$ is a hydrogen or halogen atom or it is a straight or branched $C_1$-$C_6$ alkyl group and pharmaceutically acceptable salts thereof.

The above use enables treatment of cell proliferative disorders caused by and/or associated with an altered Cdc7 kinase activity.

In a preferred embodiment of the use described above, the cell proliferative disorder is cancer.

Specific types of cancer that may be treated include carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratoxanthoma, thyroid follicular cancer, and Kaposi's sarcoma.

The present invention also provides a pyridylpyrrole derivative which is represented by formula (I)

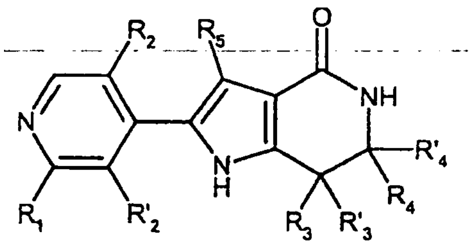

(I)

wherein
$R_1$ is a hydrogen atom, amino, $C_3$-$C_7$ cycloalkylamino ;
$R_2$ and $R'_2$ are, each independently, a hydrogen or halogen atom or a straight or branched $C_1$-$C_6$ alkyl group; or, taken together with the pyridine bond to which they are linked, $R_1$ and $R'_2$ may form a divalent -NH-CH=CH- group;
$R_3$, $R'_3$, $R_4$ and $R'_4$ are, each independently, a hydrogen atom or a group selected from straight or branched $C_1$-$C_6$ alkyl, $C_3$-$C_6$ cycloalkyl, heterocyclyl, aryl, cycloalkyl-alkyl, heterocyclyl-alkyl or aryl-alkyl; or $R_3$ and $R_3$'or $R_4$ and $R_4$', taken together, form a $C_3$-$C_6$ cyclic alkyl group;
$R_5$ is a hydrogen or halogen atom or it is a straight or branched $C_1$-$C_6$ alkyl group and pharmaceutically acceptable salts thereof.
provided that the compound is not 2-(2-aminopyridin-4-yl)-1,5,6,7-tetrahydro-4H-pyrrolo[3,2-c]pyridin-4-one.

A more complete appreciation of the invention and many of the attendant advantages thereof will be readily obtained as the same becomes better understood by reference to the following detailed description.

## DETAILED DESCRIPTION OF THE INVENTION

Several heterocyclic compounds are known in the art as protein kinase inhibitors.

Among them are, for instance, pyrrolo-pyrazoles disclosed in WO 02/12242; tetrahydroindazoles disclosed in WO 00/69846; pyrrolo-pyridines disclosed in WO 01/98299; aminophthalazinones disclosed in WO 03/014090 and aminoindazoles disclosed in WO 03/028720.

In addition, pyridylpyrrole derivatives endowed with mitogen activated protein kinase-activated protein kinase-2 Inhibitory activity are disclosed in the published PCT International Patent Application WO 04/058762 (published July 15, 2004), claiming priority of U.S. Serial No. 60/434,962, filed in December 12, 2002.

Among the compounds therein disclosed are, in particular, pyridylpyrroles which are substituted by aryl- or aryl-alkenyl- groups at the pyridine moiety; pyridylpyrroles being substituted by amino groups or halogen atoms at this same pyridine ring are also therein disclosed as synthetic intermediates. Among these latter intermediate compounds are, for instance, 2-(2-aminopyridin-4-yl)-1,5,6,7-tetrahydro-4H-pyrrolo[3,2-c]pyridin-4-one and 2-(2-chloropyridin-4-yl)-1,5,6,7-tetrahydro-4H-pyrrolo[3,2-c]pyridin-4-one.

Interestingly, the compounds of the invention fall within the broad general formula disclosed in the aforementioned patent application US 60/434962 but are not specifically exemplified therein.

The compounds of formula (I) of the invention may have asymmetric carbon atoms and may therefore exist as individual optical isomers, as racemic admixtures or as any other admixture including a majority of one of the two optical isomers, which are all to be intended as comprised within the scope of the present invention.

Likewise, the use as an antitumor agent of all the possible isomers and their admixtures of the compounds of formula (I) are also within the scope of the present invention.

In cases when compounds may exist in tautomeric forms, for instance keto-enol tautomers, each tautomeric form is contemplated as being included within this invention whether existing in equilibrium or predominantly in one form.

In the present description, unless otherwise specified, with the term straight or branched $C_1$-$C_6$ alkyl we intend any of the groups such as methyl, ethyl, n-propyl, isopropyl, n-butyl, isobutyl, tert-butyl, sec-butyl, n-pentyl, n-hexyl.

With the term amino we intend an -$NH_2$ group whilst the term arylamino comprises any group -NH-aryl, wherein aryl is as defined below.

With the term aryl we intend any carbocyclic or heterocyclic hydrocarbon with from 1 to 2 ring moieties, either fused or linked to each other by single bonds, wherein at least one of the rings is aromatic. If present, any aromatic heterocyclic hydrocarbon also referred to as heteroaryl group, comprises a 5 to 6 membered ring with from 1 to 3 heteroatoms selected among N, O or S.

Examples of aryl groups according to the invention are, for instance, phenyl, biphenyl, α- or β-naphthyl, dihydronaphthyl, thienyl, benzothienyl, furyl, benzofuranyl, pyrrolyl, imidazolyl, pyrazolyl, thiazolyl, isothiazolyl, oxazolyl, isoxazolyl, pyridyl, pyrazinyl, pyrimidinyl, pyridazinyl, indolyl, isoindolyl, purinyl, quinolyl, isoquinolyl, dihydroquinolinyl, quinoxalinyl, benzodioxolyl, indanyl, indenyl, triazolyl.

With the term $C_3$-$C_6$ cycloalkyl we intend any group such as cyclopropyl, cyclobutyl, cyclopentyl and cyclohexyl.

With the term heterocyclyl we intend any 5 or 6 membered heterocyclic ring comprising from 1 to 3 heteroatoms selected among N, O or S. Clearly, if the said heterocycle or heterocyclyl group is an aromatic heterocycle, also referred to as heteroaryl, it is encompassed by the above definition given to aryl groups.

As such, besides the above aromatic heterocycles, the term heterocyclyl also encompasses saturated or partially unsaturated heterocycles such as, for instance, pyrroline, pyrrolidine, imidazoline, imidazolidine, pyrazoline, pyrazolidine, piperidine, piperazine, morpholine.

From the above, it is clear to the skilled person that any group which name is identified as a composite name such as, for instance, cycloalkyl-alkyl, heterocyclyl-alkyl, arylalkyl and the like, have all to be intended as construed by the moieties from which they derive. In this respect, as an example, any group which is identified as an arylalkyl has to be intended as an alkyl group which is further substituted by aryl, wherein both aryl and alkyl are as above defined. Clearly when $R_3$ and $R_3$' or $R_4$ and $R_4$', taken together, form a $C_3$-$C_6$ cyclic alkyl group, the compound is referred to as spiro derivative.

Clearly, when $R_1$ and $R'_2$ are linked together so as to form a divalent -NH-CH=CH- group, fused bicyclic ring systems are obtained as having the following formula, still being an object of the invention:

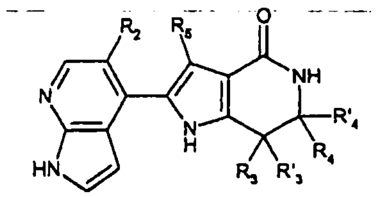

Pharmaceutically acceptable salts of the compounds of formula (I) include the acid addition salts with inorganic or organic acids such as, for instance, nitric, hydrochloric, hydrobromic, sutfuric, perchloric, phosphoric, acetic, trifluoroacetic, propionic, glycolic, lactic, oxalic, malonic, malic, maleic, tartaric, citric, benzoic, cinnamic, mandelic, methanesulphonic, isethionic and salicylic acid.

A preferred class of compounds of the invention is represented by the derivatives of formula (I) wherein at least one of $R_3$ and $R'_3$ is a hydrogen atom.

Another preferred class of compounds of the invention is represented by the derivatives of formula (I) wherein at least one of $R_4$ and $R'_4$ is a hydrogen atom.

Still more preferred compounds of the invention, within the above classes, are the derivatives of formula (I) wherein $R_1$ is hydrogen and wherein $R_2$ and $R'_2$ are, each independently, hydrogen or halogen atoms and $R_5$ is a hydrogen atom or a methyl group.

For a reference to any specific compound of formula (I) of the invention, optionally in the form of a pharmaceutically acceptable salt, see the experimental section and claims.

The compounds of formula (I) and the pharmaceutically acceptable salts thereof may be obtained by a process comprising:

a) reacting the Meldrum's acid of formula (II) with a suitable aminoacid derivative of formula (III) so as to obtain a compound of formula (IV)

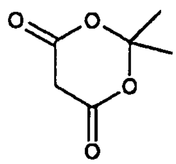

(II)

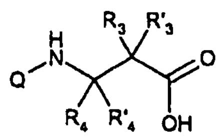

(III)

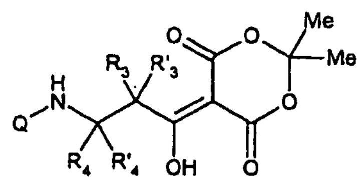

(IV)

wherein Q is a suitable nitrogen protecting group, most preferably a t-butoxycarbonyl (Boc), and $R_3$, $R'_3$, $R_4$ and $R'_4$ are as above defined;

b) heating the compound of formula (IV) in the presence of ethanol so as to obtain a compound of formula (V).

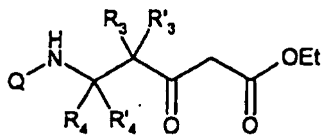

(V)

c) reacting the compound of formula (V) with a pyridine derivative of formula (VI) and treating the resulting intermediate with ammonium acetate, so as to obtain a compound of formula (VII)

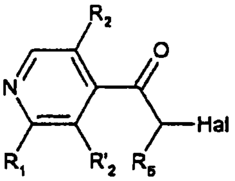

(VI)

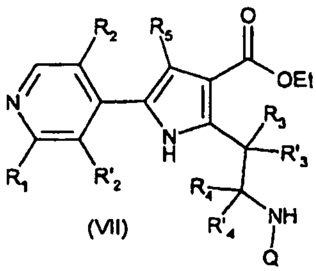

(VII)

wherein $R_1$, $R_2$, $R_3$, $R'_3$, $R_4$, $R'_4$ are as above defined, $R_5$ is a hydrogen atom or a straight or branched $C_1$-$C_6$ alkyl group, Q is the aforementioned nitrogen protecting group and Hal represents a suitable halogen atom;

d) deprotecting the compound of formula (VII) by removing the Q group in a suitable manner, e.g. by acidic treatment when Q is t-butoxy carbonyl so as to obtain a compound of formula (VIII)

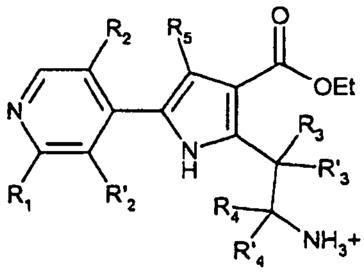

(VIII)

e) refluxing the compound of formula (VIII) in the presence of a base so as to obtain the desired compound of formula (I) and, optionally, converting it into another compound of formula (I) and/or into a pharmaceutically acceptable salt thereof.

In alternative, the product (V) may be synthesized reacting the aminoacid derivative of formula (III) with ethyl potassium malonate in the presence of 1,1'-carbonyldiimidazole and magnesium chloride.

The above process is an analogy process which can be carried out according to methods well known in the art.

According to step (a) of the process, the Meldrum's acid of formula (II) is reacted with the aminoacid derivative of formula (III) in the presence of a base, for instance dimethylaminopyridine (DMAP), and of a suitable solvent such as dichloromethane (DCM).
The reaction is carried out in the presence of a carbodiimide such as N,N'-dicyclohexylcarbodiimide at a temperature of about 0°C and for a time varying from about 2 hours to about 24 hours.

According to step (b) of the process, the crude material of formula (IV) being obtained in step (a) is dissolved in ethanol and heated at a temperature ranging from about 50°C to refluxing temperature, for a time of about 2 hours to about 12 hours, thus affording the compound of formula (V).

In the alternative route to obtain the compound of formula (V), the compound of formula (III) is reacted with potassium malonate in the presence of 1,1'-carbonyldiimidazole and magnesium chloride. In this instance, to a solution of (III), in anhydrous tetrahydrofuran (THF), 1,1'-carbonyldiimidazole is added; the solution is left shaking 2 hours and ethyl potassium malonate and magnesium chloride are added. The temperature is then brought to a suitable value, that is, from 40 to 60 °C. Preferred is 45 °C. Stirring is carried out for a suitable time from 4 to 18 h.

According to step (c) of the process, the compound of formula (V) is reacted with a suitable pyridine derivative of formula (VI), in the presence of sodium hydride and, successively, of ammonium acetate, in a suitable solvent such as, for instance, tetrahydrofuran so as to obtain a compound of formula (VII). Preferably, within the compounds of formula (VI), Hal represents a bromine or chlorine atom. In this instance to a solution of (V) in dry THF, sodium hydride is added; stirring is carried out for 30 min and a suitable pyridine derivative (VI) is added. The reaction is carried out at a temperature of about 0°C and for a time varying from about 1 hour to about 6 hours. To the crude material obtained ammonium acetate in ethanol is added. Stirring at room temperature is carried out for a suitable time from 5 to 24 hours.

The thus obtained compound of formula (VII) is then deprotected at the nitrogen atom, in step (d) of the process,

through acidic treatment, so as to obtain the corresponding amino derivative of formula (VIII) in the form of an acid addition salt.

The reaction is carried out according to conventional methods in the presence of a suitable acid such as, for instance, hydrochloric or trifluoroacetic acid and of a suitable solvent, for instance, tetrahydrofuran, dioxane or the like. Stirring at room temperature is maintained for a suitable period of time.

According to step (e) of the process, the compound of formula (VIII) is then converted into the desired compound of formula (I) by treatment with a base, for instance sodium carbonate, in the presence of a lower alcohol such as ethanol. The reaction is carried out at refluxing temperature for a time varying from about 12 hours to about 24-48 hours.

The compounds of formula (II), (III) and (VI), as well as any other reactant of the process, are known or, if not commercially available per se, they can be easily prepared according to known methods.

As an example, the pyridine derivatives of formula (VI) may be prepared by halogenating, e.g. brominating or chlorinating, a suitable pyridine-ethanone derivative, according to the following pathway:

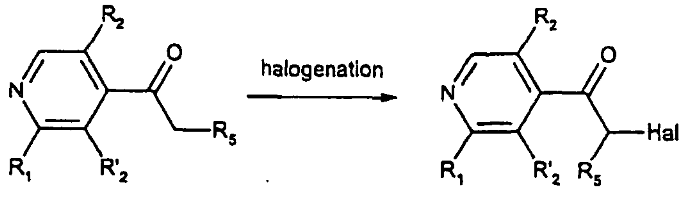


(VI)

The reaction occurs by working under conventional methods, for instance in the presence of bromine and in a suitable solvent such as a mixture of acetic and hydrobromic acid, for a time varying between about 1 hour and about 24 hours. Alternatively, a suitably activated pyridine derivative, e.g. an enolether or silylether, can be reacted with a halogen source, for instance N-bromo-succinimide (NBS), in a suitable solvent, such as tetrahydrofuran/water mixtures. Among the suitable pyridine-ethanone derivatives subdued to halogenation we consider, for instance, 1-pyridin-4-ylethanone, 1-pyridin-4-ylpropan-1-one, the intermediate 1-(2-chloropyridin-4-yl)ethanone and 1-(3-fluoropyridin-4-yl)ethanone.

1-(2-Chloropyridin-4-yl)ethanone can be easily prepared according to well known methods, for example reacting commercial 2-chloroisonicotinonitrile with a methylmagnesium halide. 1-(3-Fluoropyridin-4-yl)ethanone can be prepared, for example, by reacting commercial 3-fluoropyridine with acetaldehyde in the presence of a base, such as, for example, lithiumdiisopropylamide (LDA) and oxidizing the so obtained 1-(3-fluoropyridin-4-yl)ethanol by means of, for instance, manganese dioxide in a suitable solvent, like toluene.

In addition, the compounds of formula (VI) wherein $R_1$ and $R'_2$ are linked together so as to form a -NH-CH=CH- group, may be also prepared according to conventional methods by starting from known 4-chloro-1H-pyrrole[2,3-b] pyridine, according to the following scheme:

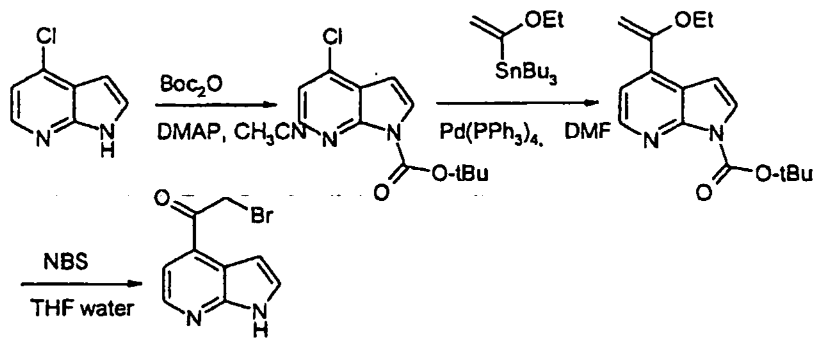


VI

4-Chloro-1H-pyrrole[2,3-b]pyridine is first protected, at the pyrrole nitrogen atom, as tert-butoxycarbonyl derivative (Boc) according to known methods, in the presence of dimethylaminopyridine and in a suitable solvent like acetonitrile. The obtained compound is then reacted with 1-(ethoxyvinyl)tributyltin, in the presence of palladium tetrakis triphenylphosphine and in dimethylformamide, so as to obtain the corresponding ethoxyvinyl derivative which, subsequently, is reacted with N-bromosuccinimide in the presence of a tetrahydrofuran/water mixture, so as to get the desired compound.

According to an alternative approach, the compounds of formula (I) may be also prepared according to the

following synthetic scheme, by reacting the above pyridine derivative of formula (VI) with a suitable piperidine-dione derivative of formula (IX) wherein Q is H or the aforementioned nitrogen protecting group, preferably tert-butoxycarbonyl or p-methoxybenzyl, p-methoxyethylbenzyl, p-methoxyphenyl group.

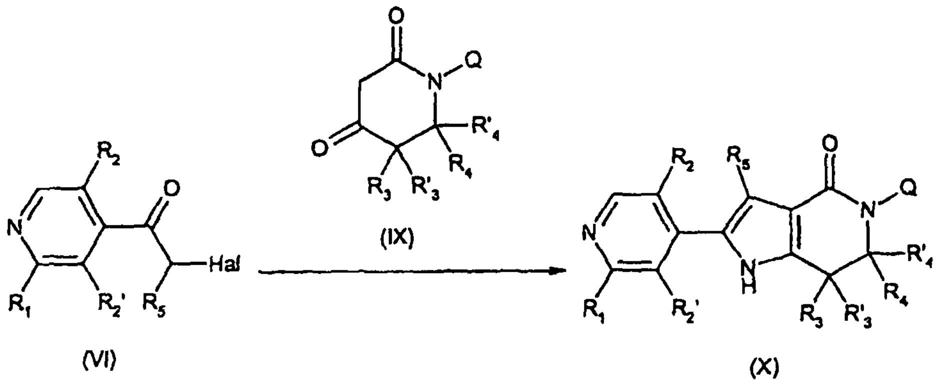

(VI) (IX) (X) ;

The reaction occurs in the presence of ammonium acetate and of a suitable solvent such as, for instance, a lower alcohol. Preferably, the reaction is carried out in the presence of ethanol by working at room temperature and for a suitable time varying from about 2 hours to about 24 hours. Optionally compound (X) can be converted into compound (I) by removal of the protecting group Q.

With this approach also compounds of formula (I), where R1 is an alkylamino or cycloalkyl amino group or an heterocycle, have been prepared starting from an intermediate of formula (X), where R1 is chloro, which was reacted with the appropriate amine or heterocycle in the presence or in the absence of a suitable solvent, but more often without solvents, and the reaction was usually carried out at temperatutes ranging from 100 to 300°C for 0.1 to 12 hours, optionally inside a microwave cavity.

Also the piperidine-dione derivative (IX) is a known compound or, alternatively, can be prepared by known methods, for instance according to the synthetic pathway below, wherein Alk stands for a suitable lower alkyl group, e.g. ethyl, and A stands for chloro or OAlk:

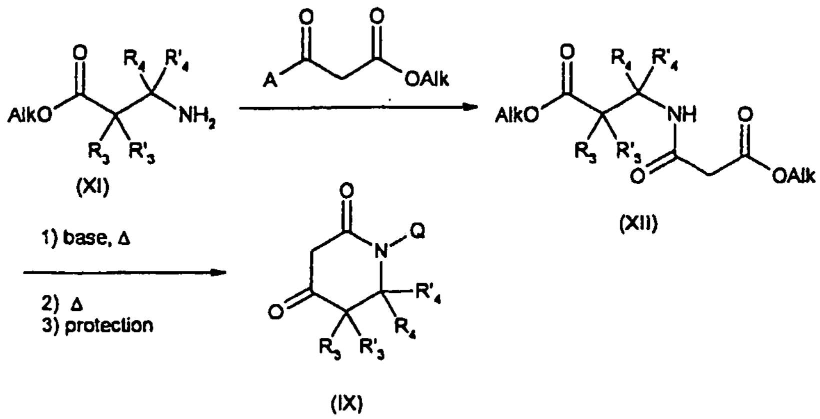


(XI) (XII) (IX)

In this respect, a suitable β-amino-carboxyester (XI) derivative wherein $R_3$, $R'_3$, $R_4$ and $R'_4$ have the above reported meanings, is reacted with dialkylmalonate or, alternatively, with 3-chloro-3-oxopropanoic acid alkyl ester, for instance, dimethylmalonate or ethyl 3-chloro-3-oxopropanoate, respectively. When A is chloro the reaction is carried out under basic conditions, for instance in the presence of triethylamine, and in a suitable solvent such as dichloromethane, at a temperature comprised between room temperature to reflux. When A is OAlk the reaction is carried out with or without basic conditions and more conveniently in the absence of solvents at reflux temperature of the dialkylmalonate.

When not commercially available, the above mentioned β-amino-carboxyester derivatives can be obtained according to well known procedures described in the literature.

The intermediate derivative thus obtained (XII) is then converted into the compound of formula (IX) in a two-steps reaction, by first reacting it under basic conditions, e.g. in the presence of sodium methylate and of a suitable solvent, preferably toluene, at refluxing temperature and for a time varying between about 2 hours and about 24 hours.

Subsequently, the product of the former step is reacted as such, without being isolated, with an acetonitrile/water/acetic acid mixture under refluxing conditions and for a time varying between about 12 hours and about 24 hours. Optionally the piperidin-dione (IX) can be protected with a suitable protecting group Q.

In the alternative, the piperidine-dione derivative (IX) can be prepared, for instance, according also to the alternative synthetic pathway below:

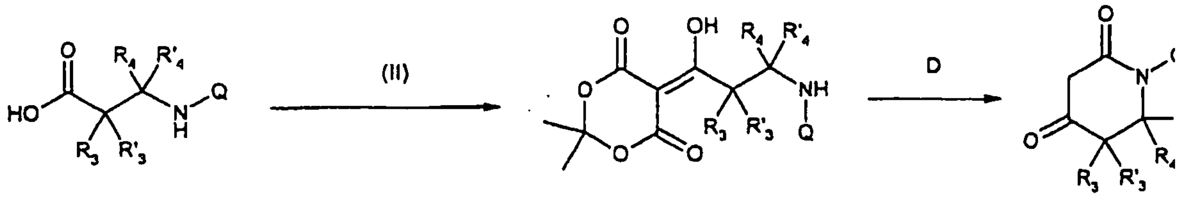


(III) (IV) (IX)

In the procedure the Meldrum's acid of formula (II) is reacted with a suitable aminoacid derivative of formula (III) so as to obtain a compound of formula (IV) wherein Q is a suitable nitrogen protecting group and $R_3$, $R'_3$, $R_4$ and $R'_4$ are as above defined. The compound of formula (IV) is then cyclized by dissolving it in a suitable solvent, for instance ethylacetate, and refluxing for a period of time from 1 to 24 hours;
or, in the alternative, the piperidine-dione derivative (IX) can be modified according to the synthetic pathway below, wherein Q stands for a suitable nitrogen-protecting group such as, in particular, tert-butoxycarbonyl, or other groups, such as p-methoxybenzyl, p-methoxyethylbenzyl, p-methoxyphenyl:

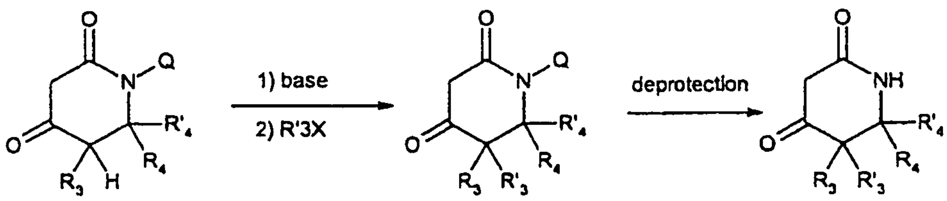


(IX) (IX) (IX)

In this respect, a suitable piperidine-dione derivative (IX) wherein $R_3$, $R_4$ and $R'_4$ and Q have the above reported meanings, is reacted with a base, for instance lithium bis(trimethylsilyl)amide (LiHMDS). The reaction is carried out in a suitable solvent such as tetrahydrofuran, at a temperature comprised between -78°C and room temperature.

The reaction mixture is then treated with a suitable alkyl halide thus obtaining another compound of formula (IX). The compound thus obtained, where Q is for instance a tert-butoxycarbonyl group, can be converted into another compound of formula (IX) by treating it with acidic conditions, e.g. in the presence of trifluoroacetic acid and of a suitable solvent, preferably dichloromethane, at room temperature and for a time comprised between about 1 hours and about 6 hours.

The final compound of formula (I) thus obtained may be then converted into another compound of formula (I) according to well-known methods in the art. As an example, the compounds of formula (I) wherein $R_5$ represents a hydrogen atom can be easily converted into the corresponding compounds of formula (I) wherein $R_5$ is a halogen atom, through conventional methods reported in the literature for the halogenation of pyrrole derivatives.

Likewise, the conversion of a compound of formula (I) into a pharmaceutically acceptable salt is easily carried out according to known methods, e.g. by contacting any free base of formula (I) with any suitable pharmaceutically acceptable acid.

From all of the above, it is clear to the skilled person that when preparing the compounds of formula (I) according to the aforementioned processes, comprehensive of any variant thereof, optional functional groups within the starting materials or the intermediates thereof and which could give rise to unwanted side reactions, need to be properly protected according to conventional techniques. Likewise, the conversion of these latter into the free deprotected compounds may be carried out according to known procedures.

By analogy, any compound of formula (I) which is susceptible of being salified can be easily converted into the corresponding acid addition salt, by working in the presence of any pharmaceutically acceptable acid, for instance

selected among those previously reported.
As it will be readily appreciated, if the compounds of formula (I) prepared according to the process described above are obtained as a mixture of isomers, they can be separated into the single isomers of formula (I), according to conventional techniques.
Conventional techniques for racemate resolution include, for instance, partitioned crystallization of diastereoisomeric salt derivatives or preparative chiral HPLC.

## PHARMACOLOGY

The compounds of formula (I) are active as protein kinase inhibitors and are therefore useful, for instance, to restrict the unregulated proliferation of tumor cells.
In therapy, they may be used in the treatment of various tumors, such as those formerly reported, as well as in the treatment of other cell proliferative disorders such as psoriasis, vascular smooth cell proliferation associated with atherosclerosis and post-surgical stenosis and restenosis and in the treatment of Alzheimer's disease.
The inhibiting activity of putative Cdc7 inhibitors and the potency of selected compounds is determined through a method of assay based on the use of Dowex resin capture technology.
The assay consists of the transfer of radioactivity labeled phosphate moiety by the kinase to an acceptor substrate. The resulting 33P-labeled product is separated from unreacted tracer, transferred into a scintillation cocktail and light emitted is measured in a scintillation counter.

### Inhibition assay of Cdc7/Dbf4 activity

The inhibition assay of Cdc7/Dbf4 activity is performed according to the following protocol.
The MCM2 substrate is trans-phosphorylated by the Cdc7/Dbf4 complex in the presence of ATP traced with $\gamma^{33}$-ATP. The reaction is stopped by addition of Dowex resin in the presence of formic acid. Dowex resin particles capture unreacted $\gamma^{33}$-ATP and drag it to the bottom of the well while $^{33}$P phosphorylated MCM2 substrate remains in solution. The supernatant is collected, transferred into Optiplate plates and the extent of substrate phosphorylation is evaluated by β counting.
The inhibition assay of Cdc7/Dbf4 activity was performed in 96 wells plate according to the following protocol.
To each well of the plate were added:

- 10 μl test compound (10 increasing concentrations in the nM to μM range to generate a dose-response curve). The solvent for test compounds contained 3% DMSO. (final concentration 1 %)

- 10 μl substrate MCM2 (6 μM final concentration), a mixture of cold ATP (2 μM final concentration) and radioactive ATP (1/5000 molar ratio with cold ATP).

- 10 μl enzyme (Cdc7/Dbf4, 2 nM final concentration) that started the reaction. The buffer of the reaction consisted in 50 mM HEPES pH 7.9 containing 15 mM $MgCl_2$, 2 mM DTT, 3 μM $NaVO_3$, 2mM glycerophosphate and 0.2mg/ml BSA.
After incubation for 60 minutes at room temperature, the reaction was stopped by adding to each well 150 μl of Dowex resin in the presence of 150 mM formic acid. After another 60 min incubation, 50 μL of suspension were withdrawn and transferred into 96-well OPTIPLATEs containing 150 μl of MicroScint 40 (Packard); after 5-10 minutes shaking the plates were read for 1 min in a Packard TOP-Count radioactivity reader.
**IC50** determination: inhibitors were tested at different concentrations ranging from 0.0005 to 10 μM. Experimental data were analyzed by the computer program Assay Explorer using the four parameter logistic equation:

$$y = bottom+(top-bottom)/(1+10^{\wedge}((logIC50-x)*slope))$$

where x is the logarithm of the inhibitor concentration, y is the response; y starts at bottom and goes to top with a sigmoid shape.
The Inhibiting activity of putative cdk/cyclin inhibitors and the potency of selected compounds may be determined through a method of assay based on the use of the SPA technology (Amersham Pharmacia Biotech).
The assay consists of the transfer of radioactivity labelled phosphate moiety by the kinase to a biotinylated substrate. The resulting 33P-labelled biotinylated product is allowed to bind to streptavidin-coated SPA beads (biotin capacity 130 pmol/mg), and light emitted was measured in a scintillation counter.

**Inhibition assay of cdk2/Cyclin A activity**

Kinase reaction: 4 μM in house biotinylated histone H1 (Sigma # H-5505) substrate, 10 μM ATP (0.1 microCi $P^{33}\gamma$-ATP), 1.1 nM Cyclic A/CDK2-complex, inhibitor in a final volume of 30 μl buffer (TRIS HCl 10 mM pH 7.5, $MgCl_2$ 10 mM, DTT 7.5 mM + 0.2 mg/ml BSA) were added to each well of a 96 U bottom. After incubation for 60 min at room temperature, the reaction was stopped by addition of 100 μl PBS buffer containing 32 mM EDTA, 500 μM cold ATP, 0.1% Triton X100 and 10mg/ml streptavidin coated SPA beads. After 20 min incubation, 110 μL of suspension were withdrawn and transferred into 96-well OPTIPLATEs containing 100 μl of 5M CsCl. After 4 hours, the plates were read for 2 min in a Packard TOP-Count radioactivity reader.

IC50 determination: inhibitors were tested at different concentrations ranging from 0.0015 to 10 μM. Experimental data were analyzed by the computer program GraphPad Prizm using the four parameter logistic equation:

$$y = bottom+(top-bottom)/(1+10^\wedge((logIC50-x)^*slope))$$

where x is the logarithm of the inhibitor concentration, y is the response; y starts at bottom and goes to top with a sigmoid shape.

**Ki calculation:**

Experimental method: Reaction was carried out in buffer (10 mM Tris, pH 7.5, 10 mM $MgCl_2$, 0.2 mg/ml BSA, 7.5 mM DTT) containing 3.7 nM enzyme, histone and ATP (constant ratio of cold/labeled ATP-1-/3000). Reaction was stopped with EDTA and the substrate captured on phosphomembrane (Multiscreen 96 well plates from Millipore). After extensive washing, the multiscreen plates were read on a top counter. Control (time zero) for each ATP and histone concentrations was measured.

**Experimental design:** Reaction velocities are measured at four ATP, substrate (histone) and inhibitor concentrations. An 80-point concentration matrix was designed around the respective ATP and substrate Km values, and the inhibitor IC50 values (0.3, 1, 3, 9 fold the Km or IC50 values). A preliminary time course experiment in the absence of inhibitor and at the different ATP and substrate concentrations allows the selection of a single endpoint time (10 min) in the linear range of the reaction for the Ki determination experiment.

**Kinetic parameter estimates:** Kinetic parameters were estimated by simultaneous nonlinear least-square regression using [Eq.1] (competitive inhibitor respect to ATP, random mechanism) using the complete data set (80 points):

$$v = \frac{Vm \bullet A \bullet B}{\alpha \bullet Ka \bullet Kb + \alpha \bullet Ka \bullet B + a \bullet Kb \bullet A + A \bullet B + \alpha \bullet \frac{Ka}{Ki} \bullet I \bullet (Kb + \frac{B}{\beta})} \quad \text{[Eq.1]}$$

where A=[ATP], B=[Substrate], I=[nhibitor], Vm= maximum velocity, Ka, Kb, Ki the dissociation constants of ATP, substrate and inhibitor respectively. α and β the cooperativity factor between substrate and ATP binding and substrate and inhibitor binding respectively.

As an example, some representative compounds of the invention were tested as formerly reported against Cdc7/Dbf4 or Cdk2, showing an Inhibitory activity, expressed as IC50 (nM), as follows:

2-(1H-pyrrolo[2,3-b]pyridin-4-yl)-1,5,6,7-tetrahydro-4H-pyrrolo[3,2-c]pyridin-4-one hydrochloride IC50 Cdc7: 16nM;
2-pyridin-4-yl-1,5,6,7-tetrahydro-4H-pyrrolo[3,2-c]pyridin-4-one hydrochloride IC50 Cdc7: 7nM;
2-(3-fluoropyridin-4-yl)-1,5,6,7-tetrahydro-4H-pyrrolo[3,2-c]pyridih-4-one hydrochloride IC50 Cdc7: 8nM;
3-methyl-2-pyridin-4-yl-1,5,6,7-tetrahydro-4H-pyrrolo[3,2-c]pyridin-4-one hydrochloride IC50 Cdc7: 3nM;
2-(1H-pyrrolo[2,3-b]pyridin-4-yl)-1,5,6,7-tetrahydro-4H-pyrrolo[3,2-c]pyridin-4-one hydrochloride IC50 Cdc7: 16nM;
(6S)-6-methyl-2-pyridin-4-yl-1,5,6,7-tetrahydro-4H-pyrrolo[3,2-c]pyridin-4-one IC50 Cdc7: 14nM;
(6R,6S)-6-benzyl-2-pyridin-4-yl-1,5,6,7-tetrahydro-4H-pyrrolo[3,2-c]pyridin-4-one IC50 Cdc7: 16nM;
(6R or 65)-6-benzyl-2-pyridin-4-yl-1,5,6,7-tetrahydro-4H-pyrrolo[3,2-c]pyridin-4-one IC50 Cdc7: 20nM;
(6R or 6S)-6-benzyl-2-pyridin-4-yl-1,5,6,7-tetrahydro-4H-pyrrolo[3,2-c]pyridin-4-one IC50 Cdc7: 9nM;
(6R,6S)-6-(2-phenylethyl)-2-pyridin-4-yl-1,5,6,7-tetrahydro-4H-pyrrolo[3,2-c]pyridin-4-one hydrochloride IC50 Cdc7: 26nM;

(7R,7S)-7-methyl-2-pyridin-4-yl-1,5,6,7-tetrahydro-4H-pyrrolo[3,2-c]pyridin-4-one hydrochloride IC50 Cdc7: 5nM;
(7R or 7S)- 7-methyl-2-pyridin-4-yl-1,5,6,7-tetrahydro-4H-pyrrolo[3,2-c]pyridin-4-one IC50 Cdc7: 4nM;
(7R or 7S)- 7-methyl-2-pyridin-4-yl-1,5,6,7-tetrahydro-4H-pyrrolo[3,2-c]pyridin-4-one IC50 Cdc7: 7nM;
(6R,6S)-6-isopropyl-2-pyridin-4-yl-1,5,6,7-tetrahydro-4H-pyrrolo[3,2-c]pyridin-4-one hydrochloride IC50 Cdc7: 9nM;
(7R,7S)-7-benzyl-2-pyridin-4-yl-1,5,6,7-tetrahydro-4H-pyrrolo[3,2-c]pyridin-4-one IC50 Cdc7: 17nM;
(6R,6S)-6-cyclopropyl-2-pyridin-4-yl-1,5,6,7-tetrahydro-4H-pyrrolo[3,2-c]pyridin-4-one IC50 Cdc7: 2nM;
(6R,6S)-6-cyclohexyl-2-pyridin-4-yl-1,5,6,7-tetrahydro-4H-pyrrolo[3,2-c]pyridin-4-one IC50 Cdc7: 8nM;
(7R,7S)-7-isopropyl-2-pyridin-4-yl-1,5,6,7-tetrahydro-4H-pyrrolo[3,2-c]pyridin-4-one IC50 Cdc7: 20nM;
(7R,7S)-7-sec-butyl-2-pyridin-4-yl-1,5,6,7-tetrahydro-4H-pyrrolo[3,2-c]pyrldin-4-one IC50 Cdc7: 3nM;
2'-pyridin-4-yl-5',6'-dihydrospiro[cyclopropane-1,7'-pyrrolo[3,2-c]pyridin]-4'(1'H)-on IC50 Cdc7: 2nM;
(7R,7S)-7-isobutyl-2-pyridin-4-yl-1,5,6,7-tetrahydro-4H-pyrrolo[3,2-c]pyridin-4-one hydrochloride IC50 Cdc7: 5nM;
(7R,7S)-7-ethyl-2-pyridin-4-yl-1,5,6,7-tetrahydro-4H-pyrrolo[3,2-c]pyridin-4-one hydrochloride IC50 Cdc7: 2nM;
7,7-dimethyl-2-pyridin-4-yl-1,5,6,7-tetrahydro-4H-pyrrolo[3,2-c]pyridin-4-one hydrochloride IC50 Cdc7: 3nM;
7,7-diethyl-2-pyridin-4-yl-1,5,6,7-tetrahydro-4H-pyrrolo[3,2-c]pyridin-4-one hydrochloride IC50 Cdc7: 7nM;
(7R,75)-2-(3-fluoropyridin-4-yl)-7-isopropyl-1,5,6,7-tetrahydro-4H-pyrrolo[3,2-c]pyridin-4-one IC50 Cdc7: 3nM;
(7R,7S)-2-(3-fluoropyridin-4-yl)-7-isobutyl-1,5,6,7-tetrahydro-4H-pyrrolo[3,2-c]pyridin-4-one IC50 Cdc7: 4nM;
(7R,7S)-2-(3-fluoropyridin-4-yl)-7-ethyl-1,5,6,7-tetrahydro-4H-pyrrolo[3,2-c]pyridin-4-one IC50 Cdc7: 1 nM and
2-[2-(cyclopentylamino)pyridin-4-yl]-1,5,6,7-tetrahydro-4H-pyrrolo[3,2-c]pyridin-4-one IC50 Cdk2: 40nM.

The compounds of the present invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors.

If formulated as a fixed dose, such combination products employ the compounds of this invention within the dosage range described below and the other pharmaceutically active agent within the approved dosage range.

Compounds of formula (I) may be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight, conditions of the patient and administration route.

For example, a suitable dosage adopted for oral administration of a compound of formula (I) may range from 10 to 500 mg per dose, from 1 to 5 times daily. The compounds of the invention can be administered in a variety of dosage forms, e.g., orally, in the form tablets, capsules, sugar or film coated tablets, liquid solutions or suspensions; rectally in the form suppositories; parenterally, e.g., intramuscularly, or through intravenous and/or intrathecal and/or intraspinal injection or infusion.

The present invention also includes pharmaceutical compositions comprising a compound of formula (I) or a pharmaceutically acceptable salt thereof in association with a pharmaceutically acceptable excipient, which may be a carrier or a diluent.

The pharmaceutical compositions containing the compounds of the invention are usually prepared following conventional methods and are administered in a suitable pharmaceutical form.

For example, the solid oral forms may contain, together with the active compound, diluents, e.g., lactose, dextrose saccharose, sucrose, cellulose, corn starch or potato starch; lubricants, e.g., silica, talc, stearic acid, magnesium or calcium stearate, and/or polyethylene glycols; binding agents, e.g., starches, arabic gum, gelatine methylcellulose, carboxymethylcellulose or polyvinyl pyrrolidone; disintegrating agents, e.g., starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be, e.g., syrups, emulsions and suspensions.

As an example, the syrups may contain, as carrier, saccharose or saccharose with glycerine and/or mannitol and sorbitol.

The suspensions and the emulsions may contain, as examples of carriers, natural gum, agar, sodium alginate, pectin, methylcellulose, carboxymethylcellulose, or polyvinyl alcohol.

The suspension or solutions for intramuscular injections may contain, together with the active compound, a

pharmaceutically acceptable carrier, e.g., sterile water, olive oil, ethyl oleate, glycols, e.g., propylene glycol and, if desired, a suitable amount of lidocaine hydrochloride. The solutions for intravenous injections or infusions may contain, as a carrier, sterile water or preferably they may be in the form of sterile, aqueous, isotonic, saline solutions or they may contain propylene glycol as a carrier.

The suppositories may contain, together with the active compound, a pharmaceutically acceptable carrier, e.g., cocoa butter, polyethylene glycol, a polyoxyethylene sorbitan fatty acid ester surfactant or lecithin.

With the aim to better illustrate the present invention, the following examples are now given.

## General methods

Flash Chromatography was performed on silica gel (Merck grade 9395, 60A). HPLC was performed on Waters X Terra RP 18 (4,6 x 50 mm, 3.5 μm) column using a Waters 2790 HPLC system equipped with a 996 Waters PDA detector and Micromass mod. ZQ single quadrupole mass spectrometer, equipped with an electrospray (ESI) ion source. Mobile phase A was ammonium acetate 5 mM buffer (pH 5.5 with acetic acid/acetonitrile 95:5), and Mobile phase B was $H_2O$/acetonitrile (5:95). Gradient from 10 to 90% B in 8 minutes, hold 90% B 2 minutes. UV detection at 220 nm and 254 nm. Flow rate 1 ml/min. Injection volume 10 μl. Full scan, mass range from 100 to 800 amu. Capillary voltage was 2.5 KV; source temp. was 120°C; cone was 10 V. Retention times (HPLC r.t.) are given in minutes at 220 nm or at 254 nm. Mass are given as m/z ratio.

When necessary, compounds have been purified by preparative HPLC on a Waters Symmetry C18 (19 x 50 mm, 5 um) column using a Waters preparative HPLC 600 equipped with a 996 Waters PDA detector and a Micromass mod. ZMD single quadrupole mass spectrometer, electron spray ionization, positive mode. Mobile phase A was water 0.01 % TFA, and Mobile phase B was acetonitrile. Gradient from 10 to 90% B in 8 min, hold 90% B 2 min. Flow rate 20 ml/min.

1H-NMR spectrometry was performed on a Mercury VX 400 operating at 400.45 MHz equipped with a 5 mm double resonance probe [1 H (15N-31 P) ID_PFG Varian].

The compounds of formula (I), having an asymmetric carbon atom and obtained as racemic mixture, were resolved by HPLC separation on chiral columns. In particular, for example, preparative columns CHIRALPACK® AD, CHIRALPACK® AS, CHIRALCELL® OJ can be used.

## Example 1

**DL-3-[(tert-butoxycarbonyl)amino]-4-phenylbutanoic acid** 2 g (11.17 mmol) of DL-3-amino-4-phenylbutanoic acid were dissolved in 50 mL of dioxane/water 1:1 and 2,3 mL of triethylamine were added. The mixture was cooled to 0°C and 2.7 g (12.38 mmol) of di-*tert*-butyl dicarbonate were added. The solution was left at room temperature overnight. The suspension was partially dried and extracted with ethyl acetate and water. The aqueous extract was acidified with 5% $NaHSO_4$ and then extracted with AcOEt three times. The organic extracts were dried over sodium sulfate and the solvent evaporated under vacuum.

About 2.5 g of DL-3-[(tert-butoxycarbonyl)amino]-4-phenylbutanoic acid were recovered.

$H^1$NMR (300 MHz, $CDCl_3$) 6 ppm 1.33 (s, 9H), 2.25-2.32 (m, 2H), 2.65 (d, 2H), 3.83-3.96 (m, 1H), 6.77 d(d, 1 H), 7.12-7.3 (m, 5H).

By working in an analogous way and by starting from the corresponding DL-beta aminoacid derivatives the following products were synthesized:

### DL-3-[(tert-butoxycarbonyl)amino]-3-cyclopropylpropanoic acid

1 H NMR (300 MHz, DMSO-D6) δ ppm -0.04 - 0.46 (m, 4 H) 0.76 - 0.97 (m, 1 H) 1.35 (s, 9 H) 2.39 (d, *J*=6.15 Hz, 2 H) 6.71 (d, *J*=9.08 Hz, 1 H) 12.04 (s, 1 H)

[M+H]+= 230

### DL-3-[(tert-butoxycarbonyl)amino]-3-cyclohexylpropanoic acid

1 H NMR (300 MHz, DMSO-D6) δ ppm 0.78 -1.77 (m, 20 H) 2.13 - 2.41 (m, 2 H) 3.52 - 3.70 (m, 1 H) 6.58 (d, J=9.38 Hz, 1 H) 11.99 (s, 1 H)

ESI (+) MS: m/z 272 (MH+).

### DL-3-[(tert-butoxycarbonyl)amino]-2-methylpropanoic acid

ESI (+) MS: m/z 204 (MH+).

**DL-3-[(tert-butoxycarbonyl)amino]-4-methylpentanoic acid**

ESI (+) MS: m/z 232 (MH+).

**DL-3-[(tert-butoxycarbonyl)amino]-4-phenylbutanoic acid**

ESI (+) MS: m/z 280 (MH+).

**Example 2**

**Preparation of ethyl 5-[(tert-butoxycarbonyl)amino]-3-oxopentanoate**

1.26 g (6.6 mmol) of N-Boc-beta-alanine were dissolved with 1 g of Meldrum's acid (6.9 mmol) and 1.28 g (10.49 mmol) of 4-dimethylaminopyridine in 70 ml of dichloromethane (DCM). The reaction mixture was cooled to 0°C and a solution of 1.58 g (7.67 mmol) of N,N'-dicyclohexylcarbodiimide in 50 mL of DCM was added dropwise. The mixture was left at 0°C overnight, during which time tiny crystals of dicyclohexylurea precipitated. After filtration, the reaction mixture was washed 3 times with an aqueous solution of 5% sodium bisulfate and one more time with brine. Organic extracts were dried over sodium sulfate and the solvent was evaporated under vacuum and then dried.

The solid was dissolved in ethanol and heated at 70°C for 6 hours. The solvent was removed and the raw product was purified by flash chromatography over silica gel thus obtaining 650 mg of the title compound as a yellow oil. H'NMR (300 MHz, CDCl3); δ ppm 1.27 (t, 3H), 1.4 (s, 9H), 2.78 (t, 2H), 3.36 (m, 2H), 3.44 (s, 2H), 4.2 (q, 2H), 5.0 (br. s., 1H).

By working in an analogous way and by starting from (3S)-3-[(tert-butoxycarbonyl)amino]butanoic acid, the following compound was prepared:

**ethyl (5S)-5-[(tert-butoxycarbonyl)amino]-3-oxohexanoate**

1 H NMR (400 MHz, DMSO-D6) δ ppm 1.03 (d, J=6.58 Hz, 3 H) 1.20 (t, J=7.13 Hz, 3 H) 1.38 (s, 9 H) 2.53 - 2.75 (m, 2 H) 3.58 (s, 2 H) 3.78 - 3.93 (m, 1 H) 4.10 (q, J=7.07 Hz, 2 H) 6.77 (d, J=7.80 Hz, 1 H).

By working in an analogous way and by starting from (3S)-3-[(tert-butoxycarbonyl)amino]-3-phenylpropanoic acid, the following compound was prepared:

**ethyl (5S)-5-[(tert-butoxycarbonyl)amino]-3-oxo-5-phenylpentanoate**

1 H NMR (400 MHz, DMSO-D6) δ ppm 1.18 (t, J=7.13 Hz, 3 H) 1.36 (s, 9 H) 2.83 - 3.05 (m, 2 H) 3.59 (s, 2 H) 4.09 (q, J=7.07 Hz, 2 H) 4.89 - 4.99 (m, 1 H) 7.16 - 7.43 (m, 6 H)

By working in an analogous way and by starting from (3R)-3-[(*tert*-butoxycarbonyl)amino]-4-methylpentanoic acid, the following compound was prepared:

**ethyl (5R)-5-[(tert-butoxycarbonyl)amino]-6-methyl-3-oxoheptanoate**

1H NMR (400 MHz, DMSO-D6) δ ppm 0.8 (d, 6 H) 1.20 (t, J=7.13 Hz, 3 H) 1.38 (s, 9 H) 1.61 - 1.72 (m, 1 H) 2.60 - 2.70 (m, 2 H) 3.59 (s, 2 H) 3.67 - 3.77 (m, 1 H) 4.10 (q, J=7.07 Hz, 2 H) 6.68 (d, J=8.78 Hz, 1 H)

By working in an analogous way and by starting from DL-3-[(tert-butoxycarbonyl)amino]-4-phenylbutanoic acid the following compound was prepared:

**ethyl 5-[(tert-butoxycarbonyl)amino]-3-oxo-6-phenylhexanoate**

1H NMR (400 MHz, DMSO-D6) δ ppm 1.18 (t, J=7.13 Hz, 3 H) 1.33 (s, 9 H) 2.62 - 2.72 (m, 4 H) 3.58 (s, 2 H) 3.96 - 4.13 (m, 4 H) 6.79 (d, J=8.41 Hz, 1 H) 7.15 - 7.33 (m, 5 H)

By-working in an analogous way and by starting from DL-3-[(tert-butoxycarbonyl)amino]-2-methylpropanoic acid the following compound was prepared:

**ethyl -5-[(tert-butoxycarbonyl)amino]-4-methyl-3-oxopentanoate**

1H NMR (400 MHz, DMSO-D6) δ ppm 0.96 (d, 3 H) 1.16 (t, 3 H) 1.34 (s, 9 H) 2.69-2-84 (m, 1 H) 2.85-3.13 (m, 1 H) 3.15-3.2 (m, 1 H) 4.62 (d, 2 H) 4.05 (q, 2H), 6.86 (s, 1H)

**Example 3**

**Preparation of ethyl DL-5-[(tert-butoxycarbonyl)amino]-6-methyl-3-oxoheptanoate**

A solution of 1 g (4.33 mmol) of 3-[(*tert*-butoxycarbonyl)amino]-4-methylpentanoic acid and 1.04 g (6.4 mmol) of 1,1'-carbonyldiimidazole in 9 mL of THF anhydrous was left shaking at room temperature 3 hours. Then, 0.817 g of magnesium chloride, 1.45 g of potassium ethyl malonate and 16mL of THF anhydrous were added. The temperature was brought to 48°C and the suspension left shaking overnight then filtered. THF was removed under vacuum and the raw product dissolved in ethyl acetate, and washed three times with an aqueous solution of 5% sodium bisulfate, three times with aq. $NaHCO_3$ and then with brine, the organic solution was dried over sodium sulfate and the solvent removed. The raw product was purified by flash chromatography over silica gel thus obtaining 1.06 g of title compound.
1 H NMR (400 MHz, DMSO-D6) δ ppm 0.8 (d, 6 H) 1.17 (t, 3 H) 1.38 (s, 9 H) 1.57 - 1.69 (m, 1 H) 2.45 - 2.65 (m, 2 H) 3.59 (s, 2 H) 3.67 - 3.77 (m, 1 H) 4.10 (q, J=7.07 Hz, 2 H) 6.68 (d, J=8.78 Hz, 1 H).
By working in an analogous way and by starting from DL 3-[(*tert*-butoxycarbonyl)amino]-3-cyclopropylpropanoic acid the following compound was prepared:

**ethyl -5-[(tert-butoxycarbonyl)amino]-5-cyclopropyl-3-oxopentanoate**

1 H NMR (400 MHz, DMSO-D6) δ ppm 0.08 - 0.42 (m, J=63.53 Hz, 4 H) 0.81 - 0.91 (m, 1 H) 1.20 (t, 3 H) 1.38 (s, 9 H) 2.65 - 2.77 (m, 2 H) 3.59 (s, 2 H) 4.09 (q, 2 H) 6.75 (s, 1 H)
By working in an analogous way and by starting from DL 3-[(*tert*-butoxycarbonyl)amino]-3-cyclohexylpropanoic acid the following compound was prepared:

**ethyl -5-[(tert-butoxycarbonyl)amino]-5-cyclohexyl-3-oxopentanoate**

1 H NMR (400 MHz, DMSO-D6) δ ppm 0.79 - 1.74 (m, 23 H) 2.44 - 2.79 (m, 2 H) 3.58 (s, 2 H) 3.66 - 3.76 (m, 1 H) 4.08 (q, J=7.19 Hz, 2 H) 6.66 (d, J=9.02 Hz, 1H).
By working in an analogous way and by starting from 2-benzyl-3-[(tert-butoxycarbonyl)amino]propanoic acid the following compound was prepared:

**ethyl 4-benzyl-5-[(tert-butoxycarbonyl)amino]-3-oxopentanoate**

ESI (+) MS: m/z 350 (MH+).

**Example 4**

**Preparation of ethyl 2-{2-[(tert-butoxycarbonyl)amino]ethyl}-5-pyridin-4-yl-1H-pyrrole-3-carboxylate**

540 mg of ethyl 5-[(tert-butoxycarbonyl)amino]-3-oxopentanoate (2.08 mmol) and 208 mg of sodium hydride (60% dispersion oil, 5.2 mmol) dissolved in 20 mL of THF were stirred 1 hour at room temperature and then cooled down to 0°C. A suspension of 735 mg (3.67 mmol) of 2-bromo-1-pyridin-4-ylethanone in 10 mL of THF was added dropwise and the mixture was stirred at 0°C for 4 hours. The resulting solution was dried and then dissolved in 30 mL of ethanol; 500 mg (8.47 mmol) of ammonium acetate were added. The solution was left stirring 5 hours and then dried. The raw product was dissolved in ethyl acetate, washed three times with brine and dried over sodium sulfate. The solvent was removed and the raw product was purified by flash chromatography over silica gel, thus obtaining 280 mg (0.78 mmol, 37%) of the title compound.
1H NMR (300 MHz, DMSO-D6) δ ppm 1.3 (t, *J*=7.0 Hz, 4 H) 1.3 (s, 9 H) 3.0 (t, *J*=7.2 Hz, 2 H) 3.2 (m, 2 H) 4.2 (q, *J*=7.0 Hz, 2 H) 7.1 (d, *J*=2.6 Hz, 1 H) 7.6 (d, *J*=6.4 Hz, 2 H) 8.5 (d, *J*=6.2 Hz, 2 H) 11.9 (s, 1 H).
HPLC retention time (RT): 4.9 min; ESI (+) MS: m/z 360 (MH+).
By working in an analogous way and by starting from the appropriate starting material the following compounds were prepared:

**ethyl 2-{(2S)-2-[(tert-butoxycarbonyl)amino]-2-phenylethyl}-5-pyridin-4-yl-1H-pyrrole-3-carboxylate**

1 H NMR (400 MHz, DMSO-D6) δ ppm 1.3 (s, 9 H) 1.3 (t, *J*=7.1 Hz, 3 H) 3.2 (dd, *J*=13.7, 9.0 Hz, 2 H) 4.2 (q, *J*=7.1 Hz, 2 H) 4.9 (m, 1 H) 7.2 (m, 7 H) 7.6 (d, *J*=6.2 Hz, 2 H) 8.5 (d, *J*=6.2 Hz, 2 H) 11.7 (s, 1 H).

**ethyl 2-{(2S)-2-[(tert-butoxycarbonyl)amino]propyl}-5-pyridin-4-yl-1H-pyrrole-3-carboxylate**

1 H NMR (300 MHz, DMSO-D6) δ ppm 1.0 (d, *J*=6.4 Hz, 3 H) 1.3 (s, 9 H) 1.3 (t, *J*=7.0 Hz, 3 H) 3.0 (m, 2H) 3.8 (m, 1 H) 4.2 (q, *J*=7.2, 2 H) 7.1 (d, *J*=2.9 Hz, 1 H) 7.6 (dd, *J*=6.2, 1.8 Hz, 2 H) 8.5 (d, *J*=6.4, 1.8 Hz, 2 H) 11.7 (s, 1 H).

**ethyl 2-{(2R)-2-[(tert-butoxycarbonyl)amino]-3-methylbutyl}-5-pyridin-4-yl-1H-pyrrole-3-carboxylate**

1H NMR (400 MHz, DMSO-D6) δ ppm 0.92 (d, J=6.71 Hz, 6 H) 1.19 (s, 9 H) 1.28-1.34 (m, 3 H) 1.67 - 1.78 (m, 1 H) 2.76 - 2.87 (m, 1 H) 3.15 - 3.23 (m, 1 H) 3.63 - 3.74 (m, 1 H) 4.17 - 4.28 (m, 2 H) 6.11 (d, J=9.39 Hz, 1 H) 7.08 (d, J=2.68 Hz, 1 H) 7.60 (dd, J=4.51, 1.59 Hz, 2 H) 8.51 (dd, J=4.51, 1.46 Hz, 2 H) 11.60 (s, 1 H)

**ethyl 2-{2-[(tert-butoxycarbonyl)amino]-3-phenylpropyl}-5-pyridin-4-yl-1H-pyrrole-3-carboxylate**

1H NMR (400 MHz, DMSO-D6) δ ppm 1.22 (s, 9 H) 1.27 (d, J=7.07 Hz, 3 H) 2.73 - 2.82 (m, 2 H) 2.91 - 2.99 (m, 1 H) 3.13 - 3.22 (m, 1 H) 4.01 - 4.25 (m, 3 H) 6.44 (d, J=8.90 Hz, 1 H) 7.09 (d, J=2.56 Hz, 1 H) 7.17 - 7.32 (m, 5 H) 7.62 (dd, J=4.63, 1.59 Hz, 2 H) 8.49 (dd, J=4.63, 1.59 Hz, 2 H) 11.71 (s, 1 H)

**ethyl 2-{2-[(tert-butoxycarbonyl)amino]-1-methylethyl}-5-pyridin-4-yl-1H-pyrrole-3-carboxylate**

1 H NMR (400 MHz, DMSO-D6) δ ppm 1.24 (d, J=7.19 Hz, 3 H) 1.30 (t, J=7.07 Hz, 3 H) 1.35 (s, 9 H) 3.78 - 3.89 (m, 1 H) 4.14 (d, J=7.13, 2 H) 6.89 (s, 1 H) 7.09 (d, J=2.80 Hz, 1 H) 7.71 (d, J=4.63 Hz, 2 H) 8.51 (d, J=4.51 Hz, 2 H) 11.48 (s, 1 H)

**ethyl 2-{2-[(tert-butoxycarbonyl)amino]-2-cyclopropylethyl}-5-pyridin-4-yl-1H-pyrrole-3-carboxylate**

1 H NMR (400 MHz, DMSO-D6) δ ppm -0.08 - 0.37 (m, 4 H) 0.85 - 0.98 (m, 1 H) 1.23 - 1.34 (m, 12 H) 2.99 - 3.38 (m, 3 H) 4.16 - 4.26 (m, 2 H) 6.56 (d, J=8.54 Hz, 1 H) 7.10 (d, J=2.80 Hz, 1 H) 7.64 (dd, J=4.63, 1.59 Hz, 2 H) 8.50 (d, 2 H) 11.73 (s, 1 H)

**ethyl 2-{2-[(tert-butoxycarbonyl)amino]-2-cyclohexylethyl}-5-pyrldin-4-yl-1H-pyrrole-3-carboxylate**

1 H NMR (400 MHz, DMSO-D6) δ ppm 1.20 - 1.22 (m, 23 H) 2.77 - 2.87 (m, 1 H) 3.15 - 3.26 (m, 1 H) 3.57 - 3.75 (m, 1 H) 4.17 - 4.28 (m, 2 H) 6.07 (d, J=9.51 Hz, 1 H) 7.09 (d, J=2.68 Hz, 1 H) 7.61 (dd, J=4.76, 1.59 Hz, 2 H) 8.51 (d, J=5.97 Hz, 2 H) 11.60 (s, 1 H)

**ethyl 2-{1-benzyl-2-[(tert-butoxycarbonyl)amino]ethyl}-5-pyridin-4-yl-1H-pyrrole-3-carboxylate**

HPLC RT 6.42 min; ESI (+) MS: m/z 450 (MH+).

**Example 5**

**Preparation of 4-[5-(2-ammonioethyl)-4-(ethoxycarbonyl)-1H-pyrrol-2-yl]pyridinium dichloride**

To a solution of 20 mg of ethyl 2-{2-[(tert-butoxycarbonyl)amino]ethyl}-5-pyridin-4-yl-1H-pyrrole-3-carboxylate, 2 mL of HCl 4 M in dioxane were added. The solution was left shaking 3 hours and then the product was dried under vacuum thus affording the title compound.
1 H NMR (400 MHz, DMSO-D6) δ ppm 1.3 (t, *J*=7.1 Hz, 3 H) 3.2 (m, 4 H) 4.3 (q, *J*=7.1 Hz, 2 H) 7.6 (d, *J*=2.6 Hz, 1 H) 8.1 (m, 3 H) 8.3 (d, *J*=6.3 Hz, 2 H) 8.7 (d, *J*=5.7 Hz, 2 H) 13.0 (s, 1 H).
HPLC RT 2.3 min; ESI (+) MS: m/z 260 (MH+).
By working in an analogous way and by starting from the appropriate starting material, the following compounds were also prepared:

**4-[5-[(2S)-2-ammoniopropyl]-4-(ethoxycarbonyl)-1H-pyrrol-2-yl]pyridinium dichloride**

1 H NMR (400 MHz, DMSO-D6) δ ppm 1.2 (d, *J*=6.6 Hz, 3 H) 1.3 (t, *J*=7.1 Hz, 3 H) 3.5 (m, 3 H) 4.3 (q, *J*=7.1 Hz, 2 H) 7.6 (d, *J*=2.7 Hz, 1 H) 8.1 (d, *J*=3.7 Hz, 3 H) 8.3 (d, *J*=6.8 Hz, 2 H) 8.7 (d, *J*=7.0 Hz, 2 H) 13.0 (s, 1 H).

**4-[5-[(2S)-2-ammonio-2-phenylethyl]-4-(ethoxycarbonyl)-1H-pyrrol-2-yl]pyridinium dichloride**

HPLC RT 2.7 min; ESI (+) MS: m/z 336 (MH+).

**ethyl 2-[(2S)-2-amino-3-methylbutyl]-5-pyridin-4-yl-1H-pyrrole-3-carboxylate dihydrochloride**

1 H NMR (400 MHz, DMSO-D6) δ ppm 0.99 (d, J=7.32 Hz, 6 H) 1.32 (d, J=7.07 Hz, 3 H) 1.86 - 1.98 (m, 1 H) 4.27 (d, J=7.07 Hz, 2 H) 7.65 (d, J=2.44 Hz, 1 H) 8.05 (br.s, 2 H) 8.31 (d, J=6.46 Hz, 2 H) 8.75 (d, J=6.83 Hz, 2 H) 13.11 (s, 1 H)

**ethyl 2-(2-amino-3-methylbutyl)-5-pyridin-4-yl-1H-pyrrole-3-carboxylate dihydrochloride ethyl 2-(2-amino-3-phenylpropyl)-5-pyridin-4-yl-1H-pyrrole-3-carboxylate dihydrochloride**

1H NMR (400 MHz, DMSO-D6) δ ppm 1.29 (t, J=7.13 Hz, 3 H) 3.13 - 3.27 (m, 2 H) 3.36 - 3.78 (m, 2 H) 4.16 - 4.25 (m, J=7.13, 1.28 Hz, 2 H) 4.28 - 4.39 (m, 1 H) 7.07 - 7.27 (m, 5 H) 7.52 (d, J=2.32 Hz, 1 H) 7.98 (br.s., 2 H) 8.39 (d, J=6.71 Hz, 2 H) 8.74 (d, J=6.83 Hz, 2 H) 12.76 (s, 1 H)

**ethyl 2-(2-amino-2-cyclohexylethyl)-5-pyridin-4-yl-1H-pyrrole-3-carboxylate dihydrochloride**

1 H NMR (400 MHz, DMSO-D6) δ ppm 0.94 - 1.95 (m, 14 H) 4.26 (d, J=7.07 Hz, 2 H) 7.65 (d, J=2.68 Hz, 1 H) 8.05 (br.s, 2 H) 8.31 (d, J=6.71 Hz, 2 H) 8.74 (d, J=6.95 Hz, 2 H) 13.09 (s, 1 H)

**ethyl 2-(2-amino-1-benzylethyl)-5-pyridin-4-yl-1H-pyrrole-3-carboxylate dihydrochloride**

1 H NMR (300 MHz, DMSO-D6) δ ppm 1.23 (d, J=7.03 Hz, 3 H) 3.20 (s, 3 H) 4.15 (q, *J*=6.74 Hz, 2 H) 4.26 - 4.39 (m, 1 H) 7.01 - 7.24 (m; 5 H) 7.54 (d, *J*=2.64 Hz, 1 H) 8.08 (s, 2 H) 8.50 (d, *J*=6.45 Hz, 2 H) 8.71 (d, *J*=6.74 Hz, 2 H) 13.12 (s, 1 H)
HPLC RT 3.4 min; ESI (+) MS: m/z 350 (MH+).

**ethyl 2-(2-aminopropyl)-5-pyridin-4-yl-1H-pyrrole-3-carboxylate dihydrochloride**

1H NMR (300 MHz, DMSO-D6) δ ppm 1.30 (d, *J*=7.03 Hz, 3 H) 1.40 (d, *J*=7.03 Hz, 3 H) 3.20 - 3.50 (m, 2 H) 4.00 - 4.12 (m, 1 H) 4.22 (q, *J*=7.03 Hz, 2 H) 7.63 (d, *J*=2.64 Hz, 1 H) 8.12 (s, 2 H) 8.55 (d, *J*=7.03 Hz, 2 H) 8.72 (d, *J*=7.03 Hz, 2 H) 13.01 (s, 1 H)
ESI (+) MS: m/z 274 (MH+).

**ethyl 2-(2-amino-2-cyclopropylethyl)-5-pyridin-4-yl-1H-pyrrole-3-carboxylate dihydrochloride**

ESI (+) MS: m/z 300 (MH+).

**Example 6**

**Preparation of 2-pyridin-4-yl-1,5,6,7-tetrahydro-4H-pyrrolo[3,2-c]pyridin-4-one hydrochloride**

To a solution of 20 mg of 2-[3-(ethoxycarbonyl)-5-pyridin-4-yl-1H-pyrrol-2-yl]ethanaminium chloride in 2 mL of ethanol, about 10 mg of potassium carbonate were added and the solution was refluxed for 16 hours. The mixture was cooled to room temperature, the solvent removed under evaporation and the raw product was purified by flash chromatography over silica gel, thus affording the title compound as a free base.

Sometimes, when required, the free base was dissolved in ethanol, treated with 4 N hydrochloric acid in dioxane and diluted with ethyl acetate until precipitation of the hydrochloride salt that was filtered, thus affording the title compound.
$^1$H NMR (DMSO-$d_e$ / 400 MHz) δ ppm 2.94 (t, 2H, J=6.83), 3.45 (t, 2H, J=6.83), 7.30 (bs, 1 H), 7.59 (s, 1H), 8.23 (d, 2H, J=7.08), 8.71 (d, 2H, J=7.08), 12.89 (bs, 1H)

By working in an analogous way and by starting from the appropriate starting materials the following compounds were also obtained:

**(65)-6-methyl-2-pyridin-4-yl-1,5,6,7-tetrahydro-4H-pyrrolo[3,2-c]pyridin-4-one**

1H NMR (400 MHz, DMSO-D6) δ ppm 1.3 (d, *J*=6.5 Hz, 3 H) 2.6 (dd, *J*=16.1, 10.5 Hz, 1 H) 2.9 (dd, *J*=16.0,

5.2 Hz, 1 H) 3.8 (m, 1 H) 7.0 (m, 2 H) 7.6 (d, *J*=6.2 Hz, 2 H) 8.5 (d, *J*=6.0 Hz, 2 H) 11.9 (s, 1 H).

**(6S)-6-phenyl-2-pyridin-4-yl-1,5,6,7-tetrahydro-4H-pyrrolo[3,2-c]pyridin-4-one**

1 H NMR (400 MHz, DMSO-D6) δ ppm 3.0 (dd, *J*=16.2, 7.2 Hz, 1 H) 3.3 (dd, *J*=16.3, 6.2 Hz, 1 H) 4.9 (dd, *J*=6.6, 2.4 Hz, 1 H) 7.1 (d, *J*=2.4 Hz, 1 H) 7.3 (m, 1 H) 7.4 (m, 4 H) 7.4 (d, *J*=2.3 Hz, 1 H) 7.6 (d, *J*=6.3 Hz, 2 H) 8.5 (d, *J*=6.1 Hz, 2 H) 11.9 (s, 1 H).

**(6R)-6-isopropyl-2-pyridtn-4-yl-1,5,6,7-tetrahydro-4H-pyrrolo[3,2-c]pyridin-4-one hydrochloride**

NMR (400 MHz, DMSO-D6) δ ppm 0.92 (dd, J=2.19, 6.83 Hz, 6H) 1.89 (m, 1H) 2.86 (m, 2H) 3.53 (m, 1 H) 7.23 (bs, 1 H) 7.58 (d, J=2.31 Hz, 1H) 8.17 (d, J=7.07 Hz, 2H) 8.70 (d, J=7.07 Hz, 2H) 12.71 (bs, 1 H)

**6-benzyl-2-pyridin-4-yl-1,5,6,7-tetrahydro-4H-pyrrolo[3,2-c]pyridin-4-one**

1 H NMR (400 MHz, DMSO-D6) δ ppm 2.58 - 3.12 (m, 4 H) 3.84 - 3.97 (m, 1 H) 6.99 - 7.05 (m, 2 H) 7.23 - 7.39 (m, 5 H) 7.57 (dd, J=6.22, 1.59 Hz, 2 H) 8.47 (dd, J=6.22, 1.46 Hz, 2 H) 11.83 (s, 1 H)

The racemate was subjected to chiral separation so to obtain the pure enantiomers.
Chiral chromatography was performed on Chiralpack® AD column (2 x 25 cm). Mobile phase was nHexane/iPropanol/Methanol 90/10/12.

Analytical conditions: Chiralpack® AD column with precolumn, mobile phase nHexane/iPropanol/Methanol 90/10/12.

**(6R or 6S)-6-benzyl-2-pyridin-4-yl-1,5,6,7-tetrahydro-4H-pyrrolo[3,2-c]pyridin-4-one**

RT 12.9 min; enantiomeric excess (e.e.) 99%

**(6R or 6S)-6-benzyl-2-pyridin-4-yl-1,5,6,7-tetrahydro-4H-pyrrolo[3,2-c]pyridin-4-one**

RT 17 min; e.e. 99%

**7-methyl-2-pyridin-4-yl-1,5,6,7-tetrahydro-4H-pyrrolo[3,2-c]pyridin-4-one**

1H NMR (400 MHz, DMSO-D6) δ ppm 1.31 (d, J=6.34 Hz, 3 H) 3.06 - 3.15 (m, 2 H) 3.45 - 3.52 (m, 1 H) 7.01 (d, J=2.44 Hz, 1 H) 7.05 (br.s., 1 H) 7.67 (dd, J=6.34, 1.71 Hz, 2 H) 8.50 (dd, J=6.22, 1.59 Hz, 2 H) 11.72 (s, 1 H)

The racemate was subjected to chiral separation so to obtain the pure enantiomers. Chiral chromatography was performed on Chiralpack® AD column (2 x 25 cm). Mobile phase was nHexane/ iPropanol/Methanol 90/10/10.
Analytical conditions: Chiralpack® AD column with precolumn, mobile phase nHexane/iPropanol/Methanol 90/10/10.

**(7R or 7S)-7-methyl-2-pyridin-4-yl-1,5,6,7-tetrahydro-4H-pyrrolo[3,2-c]pyridin-4-one**

RT 10.3 min; e.e. 99%

**(7R or 7S)-7-methyl-2-pyridln-4-yl-1,5,6,7-tetrahydro-4H-pyrrolo[3,2-c]pyridin-4-one**

RT 11.8 min; e.e. 94%

**6-isopropyl-2-pyridin-4-yl-1,5,6,7-tetrahydro-4H-pyrrolo[3,2-c]pyrldin-4-one hydrochloride**

1 H NMR (400 MHz, DMSO-D6) δ ppm 0.93 (d, J=6.83 Hz, 3 H) 0.94 (d, J=6.95 Hz, 3 H) 1.82 - 1.96 (m, 1 H) 2.80 (dd, J=16.58, 9.51 Hz, 1 H) 2.91 (dd, J=16.71, 5.73 Hz, 1 H) 3.51 - 3.60 (m, 1 H) 7.22 (s, 1 H) 7.57 (d, J=2.44 Hz, 1 H) 8.17 (d, J=7.07 Hz, 2 H) 8.70 (d, J=7.07 Hz, 2 H) 12.70 (s, 1 H)

The racemate was subjected to chiral separation so to obtain the pure enantiomers. Chiral chromatography was performed on Chiracell® OJ column (5 x 50cm). Mobile phase was n-Heptane/ Ethanol 75/25.

Analytical conditions: Chiralcell® OJ column with precolumn, mobile phase Heptane/ Ethanol 75/25.

The correct absolute configurations were assigned by comparison with (6R) enantiomer reported above and obtained by stereospecific synthesis.

**(6R)-6-isopropyl-2-pyridin-4-yl-1,5,6,7-tetrahydro-4H-pyrrolo[3,2-c]pyridin-4-one**

RT 11.8 min; e.e. 98%

**(6S)-6-isopropyl-2-pyridin-4-yl-1,5,6,7-tetrahydro-4H-pyrrolo[3,2-c]pyridin-4-one**

RT 8.7 min; e.e. 99%

**7-benzyl-2-pyridin-4-yl-1,5,6,7-tetrahydro-4H-pyrrolo[3,2-c]pyridin-4-one**

1H NMR (400 MHz, DMSO-D6) δ ppm 2.67 - 2.77 (m, 2 H)3.05-3.12 (m, 1 H) 6.99 - 7.02 (br.s, 1 H) 7.04 (d, J=2.44 Hz, 1 H) 7.23 - 7.40 (m, 5 H) 7.67 (dd, J=6.22, 1.59 Hz, 2 H) 8.52 (d, J=6.10 Hz, 2 H) 11.87 (s, 1 H)

**6-cyclopropyl-2-pyridin-4-yl-1,5,6,7-tetrahydro-4H-pyrrolo[3,2-c]pyridin-4-one**

1 H NMR (400 MHz, DMSO-D6) δ ppm 0.26 - 0.52 (m, 4 H) 0.94 - 1.09 (m, 1 H) 2.78 - 3.08 (m, 3 H) 7.03 (d, J=2.44 Hz, 1 H) 7.07 (d, J=1.10 Hz, 1 H) 7.62 (d, J=6.22 Hz, 2 H) 8.50 (d, J=6.22 Hz, 2 H) 11.92 (s, 1 H)

The racemate was subjected to chiral separation so to obtain the pure enantiomers. Chiral chromatography was performed on Chiracell® OJ column (5 x 50cm). Mobile phase was n-Heptane/ Ethanol/Methanol 75/20/5. Analytical conditions: Chiralcell® OJ column, mobile phase was n-Heptane/ Ethanol/Methanol 75/20/5.

**(6R or 6S)-6-cyclopropyl-2-pyridin-4-yl-1,5,6,7-tetrahydro-4H-pyrrolo[3,2-c]pyridin-4-one**

RT 6.3 min; e.e. 99%

**(6R or 6S)-6-cyclopropyl-2-pyridin-4-yl-1,5,6,7-tetrahydro-4H-pyrrolo[3,2-c]pyridin-4-one**

RT 8.6 min; e.e. 96%

**6-cyclohexyl-2-pyridln-4-yl-1,5,6,7-tetrahydro-4H-pyrrolo[3,2-c]pyridin-4-one**

1 H NMR (400 MHz, DMSO-D6) δ ppm 0.98 - 1.80 (m, 11 H) 2.70 - 2.89 (m, 2 H) 3.44 - 3.54 (m, 1 H) 6.93 (s, 1 H) 6.99 (d, 1 H) 7.60 (dd, J=6.22, 1.59 Hz, 2 H) 8.48 (dd, J=6.10, 1.46 Hz, 2 H) 11.87 (s, 1 H)

## Example 7

### Preparation of 6,6-dimethyl-2,4-dioxopiperldine

A solution of ethyl 3-methylbut-2-enoate (1 g, 7.8 mmol) in anhydrous ethanol (12 mL) was cooled to -20 °C and saturated with gaseous ammonia. The tube was sealed and kept at 90°C for 24 hours. The reaction was cooled to room temperature, bubbled with nitrogen to eliminate the residual ammonia and treated with a 4 N solution of HCl in dioxane (1.9 mL). After 30 minute stirring, the mixture was evaporated under reduced pressure to give ethyl 3-amino-3-methylbutanoate hydrochloride as a grey solid (1.19 g, Y=84%).

$^1$H NMR ($CDCl_3$ / 400 MHz) δ ppm 1.2 (t, 3H), 1.26 (s, 6H), 2.65 (s, 2H), 4.1 (q, 2H), 8.27 (bs, 3H).

Ethyl 3-amino-3-methylbutanoate hydrochloride (0.87 g, 4.79 mmol) was suspended on methylene chloride (12 mL) and triethylamine (1.4 mL, 2.1 eq.). The mixture was cooled to 0°C and treated dropwise with ethyl 3-chloro-3-oxopropanoate (0.64 mL, 1.05 eq.). The reaction was kept at room temperature for 2 hours, diluted with methylene chloride, washed with 1 N HCl and then with 5% $NaHCO_3$, dried over $Na_2SO_4$ and evaporated to dryness to obtain ethyl 3-[(3-ethoxy-3-oxopropanoyl)amino]-3-methylbutanoate (1.2g, Y=97%) as a red oil.

$^1$H NMR (DMSO-$d_6$ / 300 MHz) δ ppm 1.11-1.21 (m, 6H), 1.29-(s, 6H), 2.71 (s, 2H), 3.14 (s, 2H), 3.95-4.15 (m, 4H), 7.75 (bs, 1 H).

To a solution of sodium ethoxide, obtained from sodium metal (0.122 g, 5.55 mmol) in anhydrous ethanol (7 mL), a solution of ethyl 3-[(3-ethoxy-3-oxopropanoyl)amino]-3-methylbutanoate (1.2 g, 4.62 mmol) in dry toluene (7 mL) was added dropwise at room temperature, under stirring. The reaction mixture was heated at 80°C for 2 hours then it was concentrated to reduced volume and the residue was dissolved in toluene (15 mL). The organic phase was extracted with water (40 mL), the aqueous phase was acidified to pH 2-3 with 1 N HCl and extracted with ethyl acetate (4 x 50 mL). The organic phase was washed with brine, dried over anhydrous sodium sulphate and concentrated to give ethyl

6,6-dimethyl-2,4-dioxopiperidine-3-carboxylate as a yellow solid (0.7 g, Y=71%) which was used for the next step without further purification.

Ethyl 6,6-dimethyl-2,4-dioxopiperidine-3-carboxylate (0.69 g, 3.23 mmol) was dissolved in acetonitrile containing 1% of water (15 mL) and the resulting solution was refluxed for 2 hours. After evaporating to dryness, the crude material was suspended in isopropyl ether, kept under vigorous stirring and filtered to give the title compound (387 mg, Y=85%) as a light brown solid.

$^1$H NMR (DMSO-$d_6$ / 300 MHz) δ ppm 1.18 (s, 6H), 2.49 (bs, 2H), 3.13 (bs, 2H), 8.13 (bs, 1H).

## Example 8

### Preparation of 5-phenylpiperldine-2,4-dione

Ethyl cyano(phenyl)acetate (14.9 g, 78.83 mmol) was dissolved in absolute ethanol (400 mL) containing 37% hydrochloric acid (40 mL). The solution was treated with 10% Pd-C (2 g) and kept under hydrogen (40 psi) in a Parr apparatus for 24 hours. The resulting mixture was filtered to remove the catalyst and evaporated to dryness under reduced pressure. The residue was taken up with ethyl acetate, kept under vigorous stirring for 15 minutes and filtered. Obtained ethyl 3-amino-2-phenylpropanoate hydrochloride (11 g, Y=60%).

$^1$H NMR (DMSO-$d_6$ / 300 MHz) δ ppm 1.11 (t, 3H), 3.05 (dd, 1H, J=12.9, 6.15), 3.42 (dd, 1H, J=12.9, 8.79), 4.07 (m, 3H), 7.35 (m, 5H), 8.15 (br, 3H)

Ethyl 3-amino-2-phenylpropanoate hydrochloride (4.38 g, 19.13 mmol) was suspended in methylene chloride (80 mL) and triethylamine (5.86 mL, 2.2 eq.). The mixture was cooled to 0°C and treated dropwise with ethyl 3-chloro-3-oxopropanoate (2.69 mL, 1.1 eq.). The reaction was kept at room temperature for one hour, diluted with methylene chloride, washed with 1 N HCl and then with 5% $NaHCO_3$, dried over $Na_2SO_4$ and evaporated to dryness. The crude material was chromatographed on silica gel, eluting with hexane/ethyl acetate 1/1, to give 3-(2-ethoxycarbonyl-acetylamino)-2-phenyl-propionic acid ethyl ester (4.24 g, Y=72%) as an oil.

$^1$H NMR ($CDCl_3$ / 300 MHz) δ ppm 1.21 (t, 3H), 1.27 (t, 3H), 3.26 (s, 2H), 3.73 (m, 2H), 3.89 (dd, 1H. J=6.16, 8.50), 4.17 (m, 4H), 7.29 (m, 6H).

Sodium (380 mg, 16.52 mmol) was dissolved in anhydrous ethanol (13 mL) and the resulting solution was treated dropwise with 3-(2-ethoxycarbonyl-acetylamino)-2-phenyl-propionic acid ethyl ester (4.23 g, 13.76 mmol) dissolved in anhydrous toluene (35 mL). The reaction was kept at 80°C for 1.5 hours. After cooling, the mixture was extracted with water. The aqueous extracts were acidified with 2 N HCl, extracted with ethyl acetate and the organic layers were collected, dried over $Na_2SO_4$ and evaporated to dryness to obtain ethyl 2,4-dioxo-5-phenylpiperidine-3-carboxylate (1.73 g, Y=48%) which was used for the next step without further purification.

Ethyl 2,4-dioxo-5-phenylpiperidine-3-carboxylate (1.73 g, 6.63 mmol) was dissolved in acetonitrile containing 1% of water (30 mL) and the resulting solution was refluxed for 2 hours. After evaporating to dryness, the crude material was chromatographed on silica gel, eluting with methylene chloride/methanol 92/8, to give the title compound (780 mg, Y=62%) as a solid.

$^1$H NMR (DMSO-$d_6$ / 300 MHz) δ ppm 3.25 (d, 1H, J=18.75) 3.42-3.70 (m,2H), 3.61 (d, 1H, J=18.75), 3.81 (dd, 1H, J=5.57, 9.67), 7.26 (m, 5H), 8.20 (bs, 1 H).

## Example 9

### Preparation of ethyl 2-(aminomethyl)-3-methylbutanoate hydrochloride

Ethyl 2-cyano-3-methylbut-2-enoate (5.0 g, 32.6 mmol) was dissolved in 320 mL of absolute EtOH. 700 mg of $PtO_2$ and 12 mL of 4M HCl were added. The reaction mixture was hydrogenated at room temperature for 5 hours (30 psi). Filtration on a celite pad and evaporation of the solvent afforded crude title compound (quantitative yield).

1 H NMR (400 MHz, DMSO-D6) δ ppm 0.90 (d, *J*=6.83 Hz, 3 H) 0.93 (d, *J*=6.83 Hz, 3 H) 1.24 (t, *J*=7.13 Hz, 3 H) 1.92 - 2.06 (m, 1 H) 2.53 - 2.60 (m, 1 H) 2.84 - 3.17 (m, 2 H) 4.05 - 4.24 (m, 2 H) 7.84 (s, 3 H)

ESI (+) MS: m/z 160 (MH+).

By working in an analogous way and by starting from the suitable cyano derivative, the following compounds were also prepared:

### ethyl 2-(aminomethyl)-3-methylpentanoate hydrochloride

$^1$H NMR (DMSO-$d_6$ / 400 MHz) δ ppm 0.80 (m, 6 H) 1.23-1.40 (2 m, 5 H) 1.76 (m, 1 H) 2.69 (m, 1 H) 2.89 (m, 1 H), 3.09 (m, 1 H) 4.14 (m, 2H) 7.82 (s, 3H)

ESI (+) MS: m/z 174 (MH+).

**ethyl 1-(aminomethyl)cyclopropanecarboxylate hydrochloride**

ESI (+) MS: m/z 144 (MH+).

**Example 10**

**Preparation of ethyl 2-{[(3-ethoxy-3-oxopropanoyl)amino]methyl}-3-methylbutanoate**

Crude ethyl 2-(aminomethyl)-3-methylbutanoate hydrochloride was dissolved in 200 mL of dry DCM and DIPEA was added (14 mL, 2.5 eq). After cooling to 0°C, ethyl 3-chloro-3oxopropanoate was added (6.3 mL, 35.4 mmol). After stirring at room temperature overnight, the reaction mixture was diluted with DCM and washed with aq. $KHSO_4$ 5% (x2), aq. $NaHCO_3$ sat. sol. (x2) and brine. The organic layer was dried over $Na_2SO_4$, filtered and evaporated to dryness. Column chromatography (hexane/EtOAc=7/3 -> 1/1) afforded 8.35 g (30.55 mmol, 93.4% yield) of target product.
1H NMR (400 MHz, DMSO-D6) δ ppm 0.89 (d, *J*=6.82 Hz, 3 H) 0.93 (d, *J*=6.83 Hz, 3 H) 1.20 (m, 6 H) 1.82 - 1.90 (m, 1 H) 2.35 (m, 1 H) 3.19 - 3.33 (2 m, 4 H) 4.06 (m, 4 H) 8.11 (t, *J*=5.12 Hz, 1 H)
ESI (+) MS: m/z 274 (MH+).
By working in an analogous way and by starting from the suitable hydrochloride derivative, the following compounds were prepared:

**ethyl 2-{[(3-ethoxy-3-oxopropanoyl)amino]methyl}-3-methylpentanoate**

$^1$H NMR (DMSO-$d_6$ / 400 MHz) δ ppm 0.90 (m, 6 H) 1.19-1.65 (3 m, 9 H) 2.47 (m, 1 H) 3.20 (m, 4H), 4.08 (m, 4H), 8.09 (m, 1 H)
ESI (+) MS: m/z 288 (MH+).

**ethyl 1-{[(3-ethoxy-3-oxopropanoyl)amino]methyl}cyclopropanecarboxylate**

ESI (+) MS: m/z 258 (MH+).

**Example 11**

**Preparation of 5-isopropylpiperidine-2,4-dione**

Crude ethyl 2-{[(3-ethoxy-3-oxopropanoyl)amino]methyl}-3-methylbutanoate (8.35 g,_30.55 mmol) was dissolved in 215 mL of dry toluene and heated to 100°C. 6.9 mL of sodium methoxide 30 wt.% solution in methanol were added (36 mmol) and the reaction mixture was refluxed for 4 hours. After cooling at room temperature, the organic phase was washed with water (x2). The aqueous layers were collected, acidified (10% HCl) and extracted with DCM (x4). The organic layers were collected and evaporated to dryness. The crude was treated with 250 mL of 10% AcOH in water and refluxed for 3 hours. The reaction mixture was neutralized with $NaHCO_3$ (~pH 7) and extracted with DCM (x5). The organic layers were collected, dried ($Na_2SO_4$), filtered and evaporated to dryness. Column chromatography (DCM/EtOH=97/3) afforded 2.35 g of target product (15.14 mmol, 49.6% yield),
1 H NMR (400 MHz, DMSO-D6) δ ppm 0.85 (d, *J*=6.83 Hz, 3 H) 0.94 (d, J=6.95 Hz, 3 H) 2.07 - 2.17 (m, 1 H) 2.25 - 2.33 (m, 1 H) 3.09 - 3.41 (m, 4 H) 8.03 (s, 1 H)
ESI (+) MS: m/z 156 (MH+).
By working in an analogous way and by starting from the suitable aminoester derivative, the following compounds were prepared:

**5-sec-butylpiperidine-2,4-dione**

$^1$H NMR (DMSO-$d_6$ / 400 MHz) δ ppm 0.87 (m, 6 H) 1.36 (m, 2 H) 1.95 (m, 1 H) 2.35 (m, 1 H) 3.34 (m, 4H), 8.02 (s, 1 H)
ESI (+) MS: m/z 170 (MH+).

**5-azaspiro[2.5]octane-6,8-dione**

1 H NMR (400 MHz, DMSO-D6) δ ppm 0.95 - 1.02 (m, 2 H) 1.09 - 1.15 (m, 2 H) 3.33 (s, 2 H) 3.42 (s, 2 H) 8.22 (s, 1 H)
ESI (+) MS: m/z 140 (MH+).

**5,5-diethylpiperidine-2,4-dione**

1 H NMR (400 MHz, DMSO-D6) δ ppm 0.77 (t, *J*=7.56 Hz, 6 H) 1.46 (q, *J*=7.68 Hz, 4 H) 3.23 (d, *J*=3.78 Hz, 2 H) 3.26 (s, 2 H) 7.98 (s, 1 H)

**Example 12**

**6-Benzylpiperidine-2,4-dione**

A mixture of beta-homophenylalanine (9.1 g, 50.94 mmol), di-tert-butyl dicarbonate (12.2 g, 56.1 mmol), dioxane (180 mL), water (18 mL) and triethylamine (8.5 mL) was stirred at RT overnight. After concentration and multiple strippings with toluene, 3-[(tert-butoxycarbonyl)amino]-4-phenylbutanoic acid was obtained as an oil and used directly in the next step. It was dissolved in dry dichloromethane (370 mL), Meldrum acid (8.1 g, 56.1 mmol) and DMAP (9.7 g, 79 mmol) were added to it, the mixture was cooled to -5°C and dicyclohexylcarbodiimide (12.6 g, 61 mmol) was added. After addition the reaction mixture was kept in refrigerator overnight. The precipitate was filtered off and washed with dichloromethane. The filtrate was diluted with ethylacetate, washed in sequence with 10% aq $KHSO_4$, water, brine then concentrated to yield crude tert-butyl 1-benzyl-3-(2,2-dimethyl-4,6-dioxo-1,3-dioxan-5-yl)-3-oxopropylcarbamate that was dissolved in ethylacetate (250 mL) and refluxed 2 h. After concentration and treatment with diisopropylether the crystallized compound was filtered and washed to give tert-butyl 2-benzyl-4,6-dioxopiperidine-1-carboxylate as a white powder in 75% overall yield.

The t-butoxycarbonyl group could be removed by acidic treatment (4M HCl in dioxane) at RT.
1 H NMR (400 MHz, DMSO-D6) δ ppm 2.32 (dd, *J*=15.73, 8.17 Hz, 1 H) 2.42 (dd, *J*=16.34, 4.76 Hz, 1 H) 2.66 - 2.74 (m, 1 H) 2.87 - 3.02 (m, 2 H) 3.25 - 3.40 (m, 1 H) 3.84 - 3.93 (m, 1 H) 7.20 - 7.36 (m, 5 H) 8.14 (s, 1 H).

By working in an analogous way the following compounds were also obtained:

**6-isopropylpiperidine-2,4-dione**

ESI (+) MS: m/z 156 (MH+).

**6-methylpiperidine-2,4-dione**

ESI (+) MS: m/z 128 (MH+).

**5,5-dimethylpiperidine-2,4-dione**

1 H NMR (300 MHz, DMSO-D6) δ ppm 1.0 (s, 6 H) 3.15 (s, 2 H) 3.25 (s, 2 H) 8.0 (s, 1 H).

**6-(2-phenylethyl)piperidine-2,4-dione**

ESI (+) MS: m/z 218 (MH+).

**Example 13**

**Preparation of 5-benzylplperidine-2,4-dione**

To a solution of tert-butyl 2,4-dioxopiperidine-1-carboxylate (324 mg, 1.5 mmol) in dry THF (10 mL), cooled to -20°C under nitrogen, lithium bis(trimethylsilyl)amide (LiHMDS) (4 mL of 1 M solution in THF) was added dropwise. After 20 min stirring, 3.0 eq of benzyl bromide were added and the solution was stirred at -20°C for 2 hours. The reaction mixture was poured into 5% aq $KHSO_4$ and extracted with DCM (x2). To the collected (200 mL) organic layers, 20 mL of TFA were added and the resulting solution was stirred at room temperature for 2 hours. After evaporation, the residue was purified by column chromatography (hexane/EtOAc 1:2) affording 150 mg of target product (0.74 mmol, 49%).
1 H NMR (400 MHz, DMSO-D6) δ ppm 2.81 (m, 1H), 3.12 (m, 2 H) 3.34 (m, 4 H), 7.23 - 7.30 (m, 5H), 7.99 (s, 1 H)
ESI (+) MS: m/z 204 (MH+).

By working in an analogous way and by using the suitable alkyl halide, the following compounds were prepared:

**5-isobutylpiperidine-2,4-dione**

1 H NMR (400 MHz, DMSO-D6) δ ppm 0.88 (m, 6H), 1.16 (m, 1H), 1.53 (m, 1H), 1.61 (m, 1 H), 3.08 (m, 1 H)

3.20 - 3.40 (m, 4 H), 8.03 (s, 1H)
ESI (+) MS: m/z 170 (MH+).

**5-ethylpiperidine-2,4-dione**

1H NMR (400 MHz, DMSO-D6) δ ppm 0.89 (t, *J*=7.56, 3H), 1.35 (m, 1H), 1.69 (m, 1H), 2.39 (m, 1 H), 3.14 - 3.38 (m, 4 H), 8.05 (s, 1 H)
ESI (+) MS: m/z 142 (MH+).

**Example 14**

**Preparation of tert-butyl 5-ethyl-2,4-dioxopiperidine-1-carboxylate**

To a solution of tert-butyl 2,4-dioxopiperidine-1-carboxylate (1.92 g, 9.0 mmol), in dry THF (65 mL) and cooled to -20°C under nitrogen, lithium bis(trimethylsilyl)amide (LiHMDS) (27 mL of 1 M solution in THF) was added dropwise. After 20 min stirring, 2.53 mL (4.9 g, 31.3 mmol) of iodoethane were added and the solution was stirred at -20°C for 2 hours. The reaction mixture was poured in 5% aq $KHSO_4$ and extracted with DCM (x2). The collected organic layers were washed with water, dried over $Na_2SO_4$ and evaporated to dryness. The residue was purified by column chromatography (n-Hexane/EtOAc 1:1) affording 1.4 g of target product (5.8 mmol, 64%).
ESI (+) MS: m/z 242 (MH+).
By working in an analogous way and by using 1-iodo-3-methylbutane, the following compound was prepared:

**tert-butyl 5-isobutyl-2,4-dioxopiperidine-1-carboxylate**

ESI (+) MS: m/z 270 (MH+).

**Example 15**

**Preparation of 2-bromo-1-pyridin-4-ylethanone hydrobromide**

To a stirred solution of 4-acetylpyridine (10 mL, 90 mmols) in glacial acetic acid (40 mL) and 48% hydrobromic acid (15 mL), bromine (4.65 mL, 90 mmols) in glacial acetic acid (10 mL) was added dropwise. After addition, the solution was stirred at room temperature overnight. The white precipitate was filtered off and washed with absolute ethanol, thus obtaining the title compound (22.2 g Y=90%) as a white solid containing traces of dibromoderivative, that was used as such in the next step.
$^1$H NMR (DMSO-$d_6$ / 300 MHz) δ ppm 5.05 (s, 2 H) 8.15 (d, 2 H) 9.0 (d, 2 H).
By working in an analogous way and by starting from 4-propionylpyridine, the following compound was prepared:

**2-bromo-1-pyridin-4-ylpropan-1-one hydrobromide**

$^1$H NMR (DMSO-$d_6$ / 400 MHz) δ ppm 1.75 (d, 3 H) 5.85 (q, 1 H) 8.1 (d, 2 H) 8.95 (d, 2 H).

**Example 16**

**Preparation of 2-bromo-1-(3-fluoropyridin-4-yl)ethanone hydrobromide**

Into a stirred solution of 3-fluoropyridine (14 g, 144.2 mmol) in anhydrous THF (150 mL), cooled to -78°C and under argon, 79.2 mL (158.6 mmol) of a 2N solution of lithiumdiisopropylamide (LDA) in n-heptane, THF, ethylbenzene, were slowly dropped in about 1h. After stirring for 2.5h a cooled solution (ca. 0°C)of acetaldehyde (8.9 mL, 158.5 mmol) in 25 mL of anhydrous THF was slowly dropped and the reaction mixture was stirred at -78°C for 1.5 h. The solution was warmed to -30°C and a solution of ammonium chloride (150g) in 700 mL of water was added. The mixture was extracted with ethylacetate (3 x 400 mL) and the organic layers were washed with brine (4 x 200 mL) and dried over sodium sulfate. After concentration the oil was crystallized with n-hexane (40 mL) and 15.6 g (76% yield) of 1-(3-fluoropyridin-4-yl)ethanol were obtained.
A mixture of 1-(3-fluoropyridin-4-yl)ethanol (10 g, 70.3 mmol) and commercial activated $MnO_2$ (8 g, 92.1 mmol) in toluene (100 mL) were refluxed until disappearance of starting material. After cooling the mixture was filtered on a bed of celite, the cake washed with toluene and the organic phases concentrated to give 3-fluoro-4-acetyl pyridine (6.9 g, 70%) that was used directly in the next step.

To a stirred solution of 3-fluoro-4-acetylpyridine (5.3 g, 38.1 mmol) in glacial acetic acid (14 mL) and 48% hydrobromic acid (5.3 mL), bromine (2 mL, 38 mmol) in glacial acetic acid (5.3 mL) was added slowly and dropwise. After addition, the solution was stirred at 60°C for 2.5 h then it was cooled down and ethylacetate (70 mL) was added. After 30' stirring the mixture was filtered and the solid was washed thoroughly with ethylacetate and dried. The title compound was obtaind In 82% yield (9.4 g).
$^1$H NMR (DMSO-$d_6$ / 300 MHz) δ ppm 4.88 (s, 2 H) 7.83 (dd, 1 H) 8.62 (dd, 1 H) 8.81 (d, 1 H).

**Example 17**

**Preparation of (7S,7R)-7-phenyl-2-pyridin-4-yl-1,5,6,7-tetrahydro-4H-pyrroio[3,2-clpyridin-4-one hydrochloride**

A solution of 5-phenylpiperidine-2,4-dione (556 mg, 2.94 mmol) and ammonium acetate (603 mg, 7.83 mmol) in ethanol (20 mL) was treated with 2-bromo-1-pyridin-4-ylethanone hydrobromide (550 mg, 1.96 mmol) at room temperature for one hour. The mixture was concentrated under reduced pressure, diluted with ethyl acetate and washed with 5% $NaHCO_3$ solution and with water. The organic layer was dried over $Na_2SO_4$ and evaporated to dryness. The crude material was chromatographed on silica gel eluted with methylene chloride/methanol 9/1 to give the desired compound as a red foamy free base solid. The free base was dissolved in ethanol, treated with 4 N hydrochloric acid in dioxane and diluted with ethyl acetate until precipitation of the hydrochloride salt that was filtered, thus affording the title compound (220 mg; Y=34%).
$^1$H NMR (DMSO-$d_6$ / 400 MHz) δ ppm 3.87 (dd, 2H, J=5.73, 11.95), 4.41 (t, 1H), 7.18-7.38 (m, 6H), 7.66 (s, 1H), 8.18 (d, 2H, J=7.08), 8.70 (d, 2H, J=7.08), 12.55 (bs, 1H).
The title compound as a racemic (7S,7R) mixture was separated through chiral column chromatography according to conventional methods, for instance by using a CHIRALPACK® AD column (2 x 25 cm) and by eluting with a mixture n-hexane:EtOH =85:15, so as to afford the desired compounds as (7R) and (7S) enantiomers, which absolute stereochemistry was however not determined:

Analytical conditions: Chiralpack® AD column with precolumn, mobile phase n-Hexane:EtOH =85:15

**(7R or 7S)-7-phenyl-2-pyridin-4-yl-1,5,6,7-tetrahydro-4H-pyrrolo[3,2-c]pyridin-4-one hydrochloride;** RT 20 min; e.e. 98%

**(7R or 7S)-7-phenyl-2-pyridin-4-yl-1,5,6,7-tetrahydro-4H-pyrrolo[3,2-c]pyridin-4-one hydrochloride.**

RT 27 min; e.e. 96%

**Example 18**

**Preparation of 6,6-dimethyl-2-pyridin-4-yl-1,5,6,7-tetrahydro-4H-pyrrolo[3,2-c]pyridin-4-one hydrochloride**

A suspension of 6,6-dimethylpiperidine-2,4-dione (225 mg, 1.6 mmol) and 2-bromo-1-pyridin-4-ylethanone hydrobromide (300 mg, 1.06 mmol) in ethanol (12 mL) was treated with ammonium acetate (329 mg, 4.27 mmol) and kept at room temperature overnight. The mixture was evaporated under reduced pressure, taken up with water (14 mL) and filtered to give 200 mg of the title compound, as a pink solid free base. The free base was dissolved in methanol (14 mL), treated with 4 N hydrochloric acid in dioxane (0.5 mL) and diluted with ethyl acetate until precipitation of the hydrochloride salt that was filtered, thus affording the title compound (192 mg, Y=64%) as a white solid.
$^1$H NMR (DMSO-$d_6$ / 400 MHz) δ ppm 1.29(s, 6H), 2.90 (s, 2H), 7.27 (s, 1H), 7.59 (s, 1H), 8.18 (d, 2H, J=7.07), 8.70 (d, 2H, J=7.07), 12.65 (bs, 1H).

**Example 19**

**Preparation of 7-isopropyl-2-pyridin-4-yl-1,5,6,7-tetrahydro-4H-pyrrolo[3,2-c]pyridin-4-one**

A suspension of 5-isopropylpiperidine-2,4-dione (1.9 g, 12.24 mmol) and 2-bromo-1-pyridin-4-ylethanone hydrobromide (2.6 g, 9.42 mmol) in ethanol (120 mL) was treated with ammonium acetate (2.9 g, 37.7 mmol) at room temperature The resulting solution was stirred overnight. The mixture was concentrated under reduced pressure, diluted with ethyl acetate and washed with NaOH 0.5 M (pH=9). The aqueous layer was extracted with ethyl acetate (x5). The collected organic layers were dried over $Na_2SO_4$ and evaporated to dryness. The crude material was chromatographed on silica gel, eluting with methylene chloride/ethanol 10/1, to give the desired compound (1.4 g, 5.48 mmol, 58.2 %).

1 H NMR (400 MHz, DMSO-D6) δ ppm 0.92 (d, *J*=6.95 Hz, 3 H) 0.94 (d, *J*=6.70 Hz, 3 H) 1.95 - 2.13 (m, 1 H) 2.69 (m, 1 H) 3.35 (m, 1 H) 3.50 (m, 1 H) 6.97 (d, *J*=3.61 Hz, 1 H) 7.00 (d, *J*=2.43 Hz, 1 H) 7.65 (d, *J*=6.22 Hz, 2 H) 8.50 (d, *J*=6.22 Hz, 2 H) 11.74 (s, 1 H)
ESI (+) MS: m/z 256 (MH+).

The title compound as a racemic (7S,7R) mixture was separated by chiral column chromatography according to conventional methods, for instance by using a CHIRALPACK® AD column and by eluting with a mixture n-heptane: ethanol=78:22, so as to afford the desired compounds as (7R) and (7S) enantiomers, which absolute stereochemistry was however not determined.

The free base was dissolved in ethanol, treated with 4 N hydrochloric acid in dioxane and diluted with ethyl acetate until precipitation of the hydrochloride salt that was filtered, thus affording the two hydrochloride enantiomers.

By working in an analogous way and by starting from the suitable piperidine-dione derivative, the following compounds were prepared:

**7-sec-butyl-2-pyridin-4-yl-1,5,6,7-tetrahydro-4H-pyrrolo[3,2-c]pyridin-4-one**

$^1$H NMR (DMSO-$d_6$ / 400 MHz) δ ppm 0.87 (m, 6 H) 1.17 -1.50 (m, 2 H) 1.89 (m, 1 H) 2.86 (m, 1 H) 3.29 - 3.54 (m, 2H), 6.97 (s, 1H), 7.02 (s, 1H), 7.68 (m, 2H), 8.51 (m, 2H), 11.72 (s, 1 H)
ESI (+) MS: m/z 270 (MH+).

**2'-pyridin-4-yl-5',6'-dihydrospiro[cyclopropane-1,7'-pyrrolo[3,2-c]pyridin]-4'(1'H)-one**

1 H NMR (400 MHz, DMSO-D6) δ ppm 0.96 - 1.04 (m, 2 H) 1.22 - 1.31 (m, 2 H) 3.27 (d, *J*=2.44 Hz, 2 H) 7.03 (d, *J*=2.44 Hz, 1 H) 7.12 (t, *J*=2.50 Hz, 1 H) 7.65 (d, *J*=6.22 Hz, 2 H) 8.49 (d, *J*=6.22 Hz, 2 H) 11.15 (s, 1 H)
ESI (+) MS: m/z 240 (MH+).

**7-isobutyl-2-pyridin-4-yl-1,5,6,7-tetrahydro-4H-pyrrolo[3,2-c]pyridin-4-one**

1 H NMR (400 MHz, DMSO-D6) δ ppm 0.93 (d, *J*=6.46 Hz, 3 H), 0.97 (d, J=6.47 Hz, 3 H), 1.45 - 1.70 (m, 3H), 2.99 (m, 1H), 3.17 (m, 1H), 3.50 (m, 1H), 7.00 (s, 1H), 7.02 (s, 1H), 7.60 (m, 2H), 8.50 (m, 2H) 11.69 (s, 1 H).
ESI (+) MS: m/z 270 (MH+).

**7-ethyl-2-pyridin-4-yl-1,5,6,7-tetrahydro-4H-pyrrolo[3,2-c]pyridin-4-one**

1 H NMR (400 MHz, DMSO-D6) δ ppm 0.96 (t, *J*=7.44 Hz, 3 H), 1.59 (m, 1 H), 1.82 (m, 1H), 2.86 (m, 1H), 3.21 (m, 1H), 3.53 (m, 1H), 7.00 (s, 1H), 7.01 (s, 1H), 7.65 (m, 2H), 8.49 (m, 2H), 11.75 (s, 1 H)
ESI (+) MS: m/z 242 (MH+).

The racemate was subjected to chiral separation so to obtain the pure enantiomers. Chiral chromatography was performed on CHIRALPACK® AS (5 x 50 cm). Mobile phase was -n-Hex/EtOH/MeOH 70:23:7

Analytical conditions: Chiralpack® AS column, mobile phase n-Hex/EtOH/MeOH 70:10:20

**(7R or 7S)-7-ethyl-2-pyridin-4-yl-1,5,6,7-tetrahydro-4H-pyrrolo[3,2-c]pyridin-4-one**

RT 5.7 min; e.e. 99.5%

**(7R or 7S)-7-ethyl-2-pyridin-4-yl-1,5,6,7-tetrahydro-4H-pyrrolo[3,2-c]pyridin-4-one**

RT 9.3 min; e.e. 97%

**6-isopropyl-2-pyridin-4-yl-1,5,6,7-tetrahydro-4H-pyrrolo[3,2-c]pyridin-4-one hydrochloride**

1 H NMR (400 MHz, DMSO-D6) δ ppm 0.93 (d, J=6.83 Hz, 3 H) 0.94 (d, J=6.95 Hz, 3 H) 1.82 - 1.96 (m, 1 H) 2.80 (dd, J=16.58, 9.51 Hz, 1 H) 2.91 (dd, J=16.71, 5.73 Hz, 1 H) 3.51 - 3.60 (m, 1 H) 7.22 (s, 1 H) 7.57 (d, J=2.44 Hz, 1 H) 8.17 (d, J=7.07 Hz, 2 H) 8.70 (d, J=7.07 Hz, 2 H) 12.70 (s, 1 H)

The racemate was subjected to chiral separation so to obtain the pure enantiomers. Chiral chromatography was performed on CHIRALCELL® OJ (5 x 50 cm). Mobile phase was n-Hex/EtOH 75:25. Correct absolute configurations were assigned by comparison with (6R) enantiomer reported in Example 6 and obtained by stereospecific synthesis.

**(6S)-6-isopropyl-2-pyridin-4-yl-1,5,6,7-tetrahydro-4H-pyrrolo[3,2-c]pyridin-4-one hydrochloride**

1H NMR (400 MHz, DMSO-D6) δ ppm 0.93 (dd, J=2.32, 6.95 Hz, 6H) 1.70 (m, 1 H) 2.86 (m, 2H) 3.63 (m, 1 H) 7.22 (bs, 1 H) 7.56 (d, J=2.32 Hz, 1 H) 8.17 (d, J=7.08 Hz, 2H) 8.70 (d, J=7.08 Hz, 2H) 12.70 (bs, 1 H)

**(6R)-6-isopropyl-2-pyridin-4-yl-1,5,6,7-tetrahydro-4H-pyrrolo[3,2-c]pyridin-4-one hydrochloride**

1H NMR (400 MHz, DMSO-D6) δ ppm 0.92 (dd, J=2.19, 6.83 Hz, 6H) 1.89 (m, 1 H) 2.86 (m, 2H) 3.53 (m, 1H) 7.23 (bs, 1H) 7.58 (d, J=2.31 Hz, 1 H) 8.17 (d, J=7.07 Hz, 2H) 8.70 (d, J=7.07 Hz, 2H) 12.71 (bs, 1H).

**6-isobutyl-2-pyridin-4-yl-1,5,6,7-tetrahydro-4H-pyrrolo[3,2-c]pyridin-4-one hydrochloride**

1H NMR (400 MHz, DMSO-D6) δ ppm 0.91 (dd, J=6.58, 10.85 Hz, 6H) 1.38 (m, 1 H) 1.53 (m, 1H) 1.76 (m, 1 H) 2.70 (dd, J= 8.42, 16.34 Hz, 1 H) 3.05 (dd, J=5.36, 16.34, 1 H) 3.74 (m, 1 H) 7.27 (bs, 1 H) 7.58 (d, J=2.32 Hz, 1 H) 8.18 (d, J=7.07 Hz, 2H) 8.71 (d, J=7.07 Hz, 2H) 12,72 (bs, 1 H)

The racemate (as N-Boc derivative) was subjected to chiral separation so to obtain the pure enantiomers. Chiral chromatography was performed on CHIRALPACK® AD (5 x 50 cm). Mobile phase was EtOH/iPrOH/n-Heptane 60:30:10.

Analytical conditions: Chiralpack® AD column with precolumn, mobile phase EtOH/iPrOH/n-Heptane 60:30:10.

**(6R or 6S)-6-isobutyl-2-pyridin-4-yl-1,5,6,7-tetrahydro-4H-pyrrolo[3,2-c]pyridin-4-one**

RT 6.3 min; e.e. 99%

**(6R or 6S)-6-isobutyl-2-pyridin-4-yl-1,5,6,7-tetrahydro-4H-pyrrolo[3,2-c]pyridin-4-one**

RT 11.3 min; e.e. 98%

**7,7-dimethyl-2-pyridin-4-yl-1,5,6,7-tetrahydro-4H-pyrrolo[3,2-c]pyridin-4-one hydrochloride**

1 H NMR (400 MHz, DMSO-D6) δ ppm 1.38 (s, 6 H) 3.20 (s, 2 H) 7.37 (s, 1 H) 7.56 (d, J=2.32 Hz, 1 H) 8.36 (d, J=6.95 Hz, 2 H) 8.72 (d, J=6.95 Hz, 2 H) 12.34 (s, 1 H)

**7,7-diethyl-2-pyridin-4-yl-1,5,6,7-tetrahydro-4H-pyrrolo[3,2-c]pyridin-4-one hydrochloride**

1H NMR (400 MHz, DMSO-D6) δ ppm 0.83 (t, *J*=7.56 Hz, 6 H) 1.69 - 1.89 (m, 4 H) 3.28 (d, *J*=2.44 Hz, 2 H) 7.31 (s, 1 H) 7.59 (d, *J*=2.32 Hz, 1 H) 8.33 (d, *J*=6.71 Hz, 2 H) 8.71 (d, *J*=7.07 Hz, 2 H) 11.99 (s, 1 H)

**6-(2-phenylethyl)-2-pyridin-4-yl-1,5,6,7-tetrahydro-4H-pyrrolo[3,2-c]pyridin-4-one hydrochloride**

1H NMR (400 MHz, DMSO-D6) δ ppm 1.77 - 2.03 (m, 2 H) 2.63 - 2.77 (m, 2 H) 2.85 (dd, *J*=16.71, 9.76 Hz, 1 H) 3.05 (dd, *J*=16.10, 5.00 Hz, 1 H) 3.62 - 3.81 (m, 1 H) 7.25 (s, 5 H) 7.42 (s, 1 H) 7.58 (s, 1 H) 8.19 (d, *J*=6.10 Hz, 2 H) 8.70 (d, *J*=6.83 Hz, 2 H) 12.70 (s, 1 H)

**6-methyl-2-pyridin-4-yl-1,5,6,7-tetrahydro-4H-pyrrolo[3,2-c]pyridin-4-one hydrochloride**

1H NMR (400 MHz, DMSO-D6) δ ppm 1.26 (d, *J*=6.46 Hz, 3 H) 2.63 - 2.73 (dd, *J*=10.00, 16.34 Hz, 1 H) 3.01 (dd, *J*=16.40, 5.06 Hz, 1 H) 3.74 - 3.86 (m, 1 H) 7.28 (s, 1 H) 7.58 (d, *J*=2.32 Hz, 1 H) 8.18 (d, *J*=6.22 Hz, 2 H) 8.70 (d, *J*=7.07 Hz, 2 H) 12.67 (s, 1 H)

**7-cyclohexyl-2-pyridin-4-yl-1,5,6,7-tetrahydro-4H-pyrrolo[3,2-c]pyridin-4-one**

ESI (+) MS: m/z 296 (MH+).

**3-methyl-2-pyridin-4-yl-1,5,6,7-tetrahydro-4H-pyrrolo[3,2-c]pyridin-4-one hydrochloride**

1 H NMR (400 MHz, DMSO-D6) δ ppm 2.66 (s, 4 H) 2.90 (t, J=6.83 Hz, 3 H) 3.29 - 3.47 (m, 2 H) 7.24 (s, 1 H) 8.00 (d, J=6.95 Hz, 2 H) 8.71 (d, J=7.19 Hz, 2 H) 12.39 (s, 1 H)

**2-(1H-pyrrolo[2,3-b]pyridin-4-yl)-1,5,6,7-tetrahydro-4H-pyrrolo[3,2-c]pyridin-4-one hydrochloride**

1 H NMR (400 MHz, DMSO-D6) δ ppm 2.95 (t, J=6.71 Hz, 2H) 3.47 (t, J=6.71 Hz, 2H) 7.10 (s, 1 H9) 7.22 (bs, 1 H) 7.29 (s, 1 H) 7.61 (bd, 1 H) 7.71 (bt, 1 H) 8.38 (d, J= 6.10 Hz) 12.26 (bs, 1H)

**6-benzyl-3-methyl-2-pyridin-4-yl-1,5,6,7-tetrahydro-4H-pyrrolo[3,2-c]pyridin-4-one hydrochloride**

1H NMR (400 MHz, DMSO-D6) δ ppm 2.66 (s, 3 H) 2.68 - 2.83 (m, 3 H) 3.02 (dd, J=13.29, 5.12 Hz, 1 H) 3.82 - 3.95 (m, 1 H) 7.23 - 7.29 (m, 4 H) 7.31 - 7.38 (m, 2 H) 7.97 (d, J=7.19 Hz, 2 H) 8.70 (d, J=7.19 Hz, 2 H) 12.30 (s, 1 H)

**6-isobutyl-3-methyl-2-pyridin-4-yl-1,5,6,7-tetrahydro-4H-pyrrolo[3,2-c]pyridin-4-one hydrochloride**

1H NMR (400 MHz, DMSO-D6) δ ppm 0.91 (dd, J=6.58, 10.48 Hz, 6H) 1.35 (m, 1 H) 1.52 (m, 1H) 1.75 (m, 1 H) 2.66 (m, 4H) 3.02 (dd, J=5.12, 16.34 Hz, 1 H) 3.69 (m, 1 H) 7.20 (bs, 1H) 7.99 (d, J=7.08 Hz, 2H) 1.86 (d, J=7.08 Hz, 2H) 12.36 (bs, 1 H)

By working in an analogous way and by starting from 2-bromo-1-(3-fluoropyridin-4-yl)ethanone hydrobromide and the suitable piperidine-dione derivative, the following compounds were prepared:

**2-(3-fluoropyridin-4-yl)-7-isopropyl-1,5,6,7-tetrahydro-4H-pyrrolo[3,2-c]pyridin-4-one**

1 H NMR (400 MHz, DMSO-D6) δ ppm 0.92 (d, *J*=6.83 Hz, 3 H) 0.95 (d, *J*=6.75 Hz, 3 H) 2.06 (m, 1 H) 2.72 (m, 1 H) 3.35 (m, 1 H) 3.51 (m, 1 H) 6.95 (t, *J*=2.93 Hz, 1 H) 7.04 (d, *J*=3.54 Hz, 1 H) 7.81 (d, *J*=5.12 Hz, 1 H) 7.81(m, 1 H) 8.41 (m, 1 H) 8.56 (d, *J*=3.54 Hz, 1 H) 11.79 (s, 1 H)
ESI (+) MS: m/z 274 (MH+).

The racemate was subjected to chiral separation so to obtain the pure enantiomers. Chiral chromatography was performed on CHIRALPACK® AS (5 x 50 cm). Mobile phase was n-Hex/EtOH/MeOH 75:18:7.

Analytical conditions: Chiralpack® AS column, mobile phase n-Hex/EtOH/MeOH 70:15:15.

**(7R or 7S)-2-(3-fluoropyridin-4-yl)-7-isopropyl-1,5,6,7-tetrahydro-4H-pyrrolo[3,2-c]pyridin-4-one**

RT 6.2 min; e.e. 99.5%

**(7R or 7S)-2-(3-fluoropyridin-4-yl)-7-isopropyl-1,5,6,7-tetrahydro-4H-pyrrolo[3,2-c]pyridin-4-one**

RT 9.4 min; e.e. 90%

**2-(3-fluoropyridin-4-yl)-6-isobutyl-1,5,6,7-tetrahydro-4H-pyrrolo[3,2-c]pyridin-4-one hydrochloride**

1 H NMR (400 MHz, DMSO-D6): δ ppm 0.91 (dd, J=6.46, 10.49 Hz, 6H) 1.38 (m, 1 H) 1.54 (m, 1 H) 1.76 (m, 1 H) 2.69 (dd, J=8.91, 16.47 Hz, 1 H) 3.03 (dd, J=5.37, 16.47 Hz, 1 H) 3.73 (m, 1 H) 7.12 (t, J=2.80 Hz, 1 H) 7.18 (bs, 1 H) 7.98 (dd, J=5.73. 7.20 Hz, 1H) 8.51 (d, J=5.73 Hz, 1 h) 8.76 (d, J=4.39 Hz, 1 H)

The racemate was subjected to chiral separation so to obtain the pure enantiomers. Chiral chromatography was performed on CHIRALCELL® OJ (5 x 50 cm). Mobile phase was n-Hex/EtOH 80:20.

Analytical conditions: Chiralcell® OJ column, with precolumn, mobile phase n-Hex/EtOH 80:20.

**(6R or 6S)-2-(3-fluoropyridin-4-yl)-6-isobutyl-1,5,6,7-tetrahydro-4H-pyrrolo[3,2-c]pyridin-4-one hydrochloride**

RT 8.3 min; e.e. 99.4%

**(6R or 6S)-2-(3-fluoropyridin-4-yl)-6-isobutyl-1,5,6,7-tetrahydro-4H-pyrrolo[3,2-c]pyridin-4-one hydrochloride**

RT 10.2 min; e.e. 95%

**2-(3-fluoropyridin-4-yl)-7-isobutyl-1,5,6,7-tetrahydro-4H-pyrrolo[3,2-c]pyridin-4-one** 1 H NMR (400 MHz, DMSO-D6) δ ppm 0.93 (d, *J*=6.41 Hz, 3 H), 0.95 (d, *J*=6.45 Hz, 3 H), 1.48 - 1.71 (m, 3H), 3.02 (m, 1H), 3.21 (m, 1H), 3.51 (m, 1H), 6.93 (m, 1H), 7.07 (s, 1H), 7.81 (m, 1H), 8.41 (m, 1H), 8.56 (d, *J*=3.41 Hz, 1 H), 11.75 (s, 1 H).
ESI (+) MS: m/z 288 (MH+).

The racemate, as Boc derivative, was subjected to chiral separation so to obtain the pure enantiomers. Chiral chromatography was performed on CHIRALPACK® AD (5 x 50 cm). Mobile phase was n-Hex/EtOH 80:20.

Analytical conditions: Chiratpack® AD column, mobile phase n-Hex/EtOH 85:15.

**(7R or 7S)-2-(3-fluoropyridin-4-yl)-7-isobutyl-1,5,6,7-tetrahydro-4H-pyrrolo[3,2-c]pyridin-4-one**

**(7R or 7S)-2-(3-fluoropyridin-4-yl)-7-isobutyl-1,5,6,7-tetrahydro-4H-pyrrolo[3,2-c]pyridin-4-one**

**2-(3-fluoropyridin-4-yl)-7-ethyl-1,5,6,7-tetrahydro-4H-pyrrolo[3,2-c]pyrldin-4-one**

1H NMR (400 MHz, DMSO-D6) δ ppm 0.96 (t, *J*=7.43 Hz, 3 H), 1.62 (m, 1 H), 1.80 (m, 1H), 2.89 (m, 1H), 3.25 (m, 1H), 3.54 (m, 1H), 6.94 (m, 1H), 7.07 (s, 1H), 7.80 (m, 1H), 8.41 (m, 1H), 8.55 (d, *J*=3.40 Hz, 1 H), 11.81 (s, 1 H) ESI (+) MS: m/z 260 (MH+).

The racemate was subjected to chiral separation so to obtain the pure enantiomers. Chiral chromatography was performed on CHIRALPACK® AD (5 x 50 cm). Mobile phase was n-Hex/iPrOH/MeOH 80:5:15

Analytical conditions: Chiralpack® AD column, mobile phase n-Hex/EtOH 80:20.

**(7R or 7S)-7-ethyl-2-(3-fluoropyridin-4-yl)-1,5,6,7-tetrahydro-4H-pyrrolo[3,2-c]pyridin-4-one**

RT 9.4 min; e.e. 99.5%

**(7R or 7S)-7-ethyl-2-(3-fluoropyridin-4-yl)-1,5,6,7-tetrahydro-4H-pyrrolo[3,2-c]pyridin-4-one**

RT 10.6 min; e.e. 99.5%

**2-(3-fluoropyridin-4-yl)-1,5,6,7-tetrahydro-4H-pyrrolo[3,2-c]pyridin-4-one hydrochloride**

1 H NMR (400 MHz, DMSO-D6) δ ppm 2.91 (m, 2H) 3.41 (m, 2H) 7.08 (bs, 1H) 7.19 (bs, 1H) 7.90 (dd, J=5.60, 7.19 Hz, 1H) 8.48 (d, J=5.00 Hz, 1H) 8.70 (d, J=4.15 Hz, 1H) 12.15 (bs, 1H)

**6-benzyl-2-(3-fluoropyridin-4-yl)-1,5,6,7-tetrahydro-4H-pyrrolo[3,2-c]pyridin-4-one hydrochloride**

1H NMR (400 MHz, DMSO-D6) δ ppm 2.63 - 3.13 (m, 4 H) 3.85 - 4.02 (m, 1 H) 7.10 - 7.15 (m, 1 H) 7.20 - 7.30 (m, 4 H) 7.32 - 7.38 (m, 2 H) 7.88 - 7.98 (m, 1 H) 8.50 (dd, J=5.49, 2.68 Hz, 1 H) 8.76 (t, J=4.69 Hz, 1 H) 12.16 (s, 1 H) By working in an analogous way and by starting from 2-bromo-1-(3-fluoropyridin-4-yl)ethanone hydrobromide and the N-protected (as Boc) piperidine-dione derivative, the following compounds were prepared:

**tert-butyl 7-ethyl-2-(3-fluoropyrldin-4-yl)-4-oxo-1,4,6,7-tetrahydro-5H-pyrrolo[3,2-c]pyridine-5-carboxylate**

1H NMR (400 MHz, DMSO-D6) δ ppm 0.99 (t, *J*=7.44 Hz, 3 H), 1.49 (s, 9H), 1.60 (m, 1 H), 1.81 (m, 1H), 2.99 (m, 1H), 3.91 (dd, *J*=4.27 Hz, *J*=13.17, 1H), 4.07 (dd, *J*=4.39 Hz, *J*=13.29, 1 H), 7.03 (m, 1H), 7.81 (m, 1H), 8.45 (m, 1H), 8.60 (d, J=3.29 Hz, 1 H), 12.07 (s, 1 H).
ESI (+) MS: m/z 360 (MH+).

**tert-butyl 7-isobutyl-2-(3-fluoropyridin-4-yl)-4-oxo-1,4,6,7-tetrahydro-5H-pyrrolo[3,2-c]pyridine-5-carboxylate**

ESI (+) MS: m/z 388 (MH+).

**Example 20**

**2-(2-imidazol-1-yl-pyridin-4-yl)-1,5,6,7-tetrahydro-pyrrolo[3,2-c]pyridin-4-one**

A mixture of 2-(2-chloro-pyridin-4-yl)-1,5,6,7-tetrahydro-pyrrolo[3,2-c]pyridin-4-one (200 mg, 0.81 mmol), and imidazole (332 mg, 4.88 mmol) was stirred at 250 °C. After 4h the mixture was cooled at room temperature and the crude residue purified by preparative reverse phase HPLC, yielding the title compound as a pale brown solid (200 mg, 88% yield).
$^1$H-NMR 400MHz (DMSO-d6): δ ppm 2.90 (t, *J* = 8.0 Hz , 2H), 3.45 (m, 2H), 7.09 (m, 1H), 7.16 (m, 1H), 7.31 (m, 1H), 7.60 (m, 1H), 8.00 (m, 1 H), 8.03 (m, 1H), 8.41 (m, 1H), 8.57 (m, 1H), 11.99 (s, 1H);
MS (ESI) 280 $(M+H)^+$.

By analogous procedure the following compounds were obtained:

**2-(2-pyrrolidin-1-yl-pyridin-4-yl)-1,5,6,7-tetrahydro-pyrrolo[3,2-c]pyridin-4-one**

$^1$H-NMR 400MHz (DMSO-d6): δ ppm 1.97 (m, 4H), 2.85 (m, 2H), 3.43 (m, 6H), 6.69 (m, 1H), 6.82 (m, 1H), 6.88 (m, 1H), 6.99 (m, 1H), 7.97 (m, 1H), 11.76 (s, 1H);
MS (ESI) 283 $(M+H)^+$.

**2-(2-pyrazol-1-yl-pyridin-4-yl)-1,5,6,7-tetrahydro-pyrrolo[3,2-c]pyridin-4-one**

$^1$H-NMR 400MHz (DMSO-d6): δ ppm 2.87 (t, *J* = 8.0 Hz , 2H), 3.43 (m, 2H), 6.61 (m, 1H), 7.09 (m, 2H), 7.62 (m, 1H), 7.87 (m, 1H), 8.18 (m, 1H), 8.36 (m, 1H), 8.63 (m, 1H), 12.20 (s, 1H);
MS (ESI) 280 $(M+H)^+$.

**2-[2-(3-amino-pyrrolidin-1-yl)-pyridin-4-yl]-1,5,6,7-tetrahydro-pyrrolo[3,2-c]pyridin-4-one**

$^1$H-NMR 400MHz (DMSO-d6): δ ppm 1.77 (m, 1H), 2.10 (m, 1H), 2.85 (t, *J* = 8.0 Hz , 2H), 3.12 (m, 1H), 3.42 (m, 4H), 3.57 (m, 2H), 6.65 (m, 1H), 6.80 (m, 1H), 6.86 (m, 1H), 6.98 (s, 1H), 7.98 (m, 1H), 11.76 (s, 1H);
MS (ESI) 298 $(M+H)^+$.

**Example 21**

**2-(2-cyclopentylamino-pyridin-4-yl)-1,5,6,7-tetrahydro-pyrrolo[3,2-c]pyridin-4-one**

2-(2-Chloro-pyridin-4-yl)-1,5,6,7-tetrahydro-pyrrolo[3,2-c]pyridin-4-one (100 mg, 0.40 mmol) and cyclopentylamine (2.5 mL) were placed in a process vial which was sealed with a teflon septum and then placed in the microwave cavity. The reaction mixture was stirred for 10 min at 250°C, thereafter diluted with ethyl acetate and washed with brine. The organic phase was dried ($MgSO_4$), and the solvent was evaporated. The residue was purified by flash chromatography (DCM-MeOH-30% $NH_4OH$, 95:5:0.5) to give the product as a white solid (42 mg, 35% yield).
$^1$H NMR (400 MHz, DMSO-d6) δ ppm 1.38 - 1.76 (m, 6 H), 1.89 - 2.01 (m, 2 H), 2.83 (t, *J*=6.89 Hz, 2 H), 3.36 - 3.46 (m, 2 H), 3.99 - 4.17 (m, 1 H), 6.32 (d, *J*=4.51 Hz, 1 H), 6.63 (s, 1 H), 6.71 - 6.77 (m, 2 H), 6.99 (t, *J*=2.38 Hz, 1 H), 7.91 (d, *J*=5.37 Hz, 1 H), 11.71 (s, 1 H).

## Claims

1. Use of a compound of formula (I) for the manufacture of a medicament for treating cell proliferative disorders caused by and/or associated with an altered protein kinase activity selected from the group consisting of cancer, Alzheimer's disease, viral infections, auto-immune diseases, neurodegenerative disorders, benign prostate hyperplasia, familial adenomatosis polyposis, neuro-fibromatosis, psoriasis, vascular smooth cell proliferation associated with atherosclerosis, pulmonary fibrosis, arthritis, glomerulonephritis and post-surgical stenosis and restenosis, wherein the compound is to be administered in an effective amount to a mammal in need thereof,

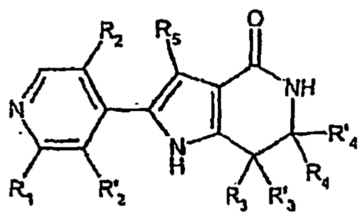

(I)

wherein
$R_1$ is a hydrogen atom, amino or $C_3$-$C_7$ cycloalkylamino;
$R_2$ and $R'_2$ are, each independently, a hydrogen or halogen atom or a straight or branched $C_r$-$C_8$ alkyl group; or, taken together with the pyridine bond to which they are linked, $R_1$ and $R'_2$ may form a divalent -NH-CH=CH- group;
$R_8$, $R'_3$, $R_4$ and $R'_4$ are, each independently, a hydrogen atom or a group selected from straight or branched $C_1$-$C_6$ alkyl, $C_3$-$C_8$ cycloalkyl, heterocyclyl, aryl, cycloalkyl-alkyl, heterocyclyl-alkyl
or aryl-alkyl; or $R_3$ and $R_3$' or $R_4$ and $R_4$', taken together, form a $C_3$-$C_6$ cycllo alkyl group;
$R_5$ is a hydrogen or halogen atom or it is a straight or branched $C_1$-$C_6$ alkyl group and pharmaceutically acceptable

salts thereof.

2. The use according to claim 1 wherein the cancer is selected from the group consisting of carcinoma, squamous cell carcinoma, hematopoietic tumors of myeloid or lymphoid lineage, tumors of mesenchymal origin, tumors of the central and peripheral nervous system, melanoma, seminoma, teralocarcinoma, osteosarcoma, xeroderma pigmentosum, keratoxanthoma, thyrold follicular cancer, and Kaposi's sarcoma.

3. The use according to claim 1, wherein the mammal in need thereof is further to be subjected to a radiation therapy or chemotherapy regimen in combination with at least one cytostatic or cytotoxic agent.

4. The use according to claim 1 wherein the mammal in need thereof is a human.

5. A compound of formula (I)

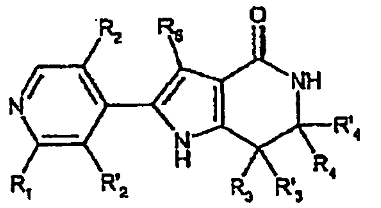

(I)

wherein
$R_1$ is a hydrogen atom, amino or $C_3$-$C_7$ cycloalkylamino;
$R_2$ and $R'_2$ are, each independently, a hydrogen or halogen atom or a straight or branched $C_1$-$C_6$ alkyl group; or, taken together with the pyridine bond to which they are linked, $R_1$ and $R'_2$ may form a divalent -NH-CH=CH- group:
$R_3$, $R'_3$, $R_4$ and $R'_4$ are, each independently, a hydrogen atom or a group selected from straight or branched $C_1$-$C_6$ alkyl, $C_3$-$C_8$ cycloalkyl, heterocyclyl, aryl, cycloalkyl-alkyl, heterocyclyl-alkyl or aryl-alkyl; or $R_3$ and $R_3$' or $R_4$ and $R_4$', taken together, form a $C_3$-$C_8$ cyclic alkyl group;
$R_5$ is a hydrogen or halogen atom or it is a straight or branched $C_1$-$C_6$ alkyl group and pharmaceutically acceptable salts thereof;.
provide that the compound is not 2-(2-aminopyridin-4-yl)-1,5,6,7-tetrahydro-4H-pyrrolo[3,2-c]pyridin-4-one.

6. A compound of formula (I) as defined in claim 5 wherein $R_3$ and $R'_3$ are both hydrogen atoms or one of them is a phenyl group and the remaining one is a hydrogen atom; and $R_1$, $R_2$, $R'_2$, $R_4$, $R'_4$ and $R_5$ are as defined in claim 5.

7. A compound of formula (I) as defined in claim 5 wherein $R_4$ and $R'_4$ are both hydrogen atoms or methyl groups or one of them is a methyl or phenyl group and the remaining one is a hydrogen atom; and $R_1$, $R_2$, $R'_2$ $R_3$, $R'_3$ and $R_5$ are as defined in claim 5.

8. A compound of formula (I) as defined in claim 5 wherein $R_1$ is hydrogen and wherein $R_2$ and $R'_2$ are, each independently, hydrogen or halogen atoms; $R_5$ is a hydrogen atom or a methyl group and $R_3$, $R'_3$, $R_4$ and $R'_4$ are as above defined.

9. A compound of formula (I) according to claim 5, optionally in the form of a pharmceutically acceptable salt thereof, selected from the group consisting of:

2-(1H-pyrrolo[2,3-b]pyridin-4yl)-1,5,6,7-tetrahydro-4H-pyrrolo[3,2-c]pyridin-4-one hydrochloride;
2-pyridin-4-yl-1,5,6,7-tetrahydro-4H-pyrrolo[3,2-c]pyridin-4-one hydrochloride;
2-(3-fluoropyridin-4-yl)-1,5.6,7-tetrahydro-4H-pyrrolo[3,2-c]pyridin-4-one hydrochloride;
3-methyl-2-pyridin-4-yl-1,5,6,7-teirahydro-4H-pyrrolo[3,2-c]pyridin-4-one hydrochloride;
2-(1H-pyrrolo[2,3-b]pyridin-4-yl)-1,5,6,7-tetrahydro-4H-pyrrolo[3,2-c]pyridin-4-one;
(6S)-6-methyl-2-pyridin-4-yl-1,5,6,7-tetrahydro-4H-pyrrolo[3,2-c]pyridin-4-one;
(6R,6S)-6-benzyl-2-pyridin-4-yl-1,5,6,7-tetrahydro-4H-pyrrolo[3,2-c]pyridin-4-one;
(6R or 6S)-6-benzyl-2-pyridin-4-yl-1,5,6,7-tetrahydro-4H-pyrrolo[3,2-c]pyridin-4-one;
(6R,6S)-6-(2-phenylethyl)-2-pyridin-4-yl-1,5,6,7-tetrahydro-4H-pyrrolo[3,2-c]pyridin-4-one hydrochloride;
(7R,7S)-7-methyl-2-pyridin-4-yl-1,5,6,7-tetrahydro-4H-pyrrolo[3,2-c]pyridin-4-one hydrochloride;

(7R or 7S)- 7-methyl-2-pyridin-4-yl-1,5,6,7-tetrahydro-4H-pyrrolo[3,2-c]pyridin-4-one;
(6R,6S)-6-isopropyl-2-pyridin-4-yl-1,5,6,7-tetrahydro-4H-pyrrolo[3,2-c]pyridin-4-one hydrochloride;
(7R,7S)-7-benzyl-2-pyridin-4-yl-1,5,6,7-tetrahydro-4H-pyrrolo[3,2-c]pyridin-4-one;
(6R,6S)-6-cyclopropyl-2-pyridin-4-yl-1,5,6,7-tetrahydro-4H-pyrrolo[3,2-c]pyridin-4-one;
(6R,6S)-6-cyclohexyl-2-pyridin-4-yl-1,5,6,7-tetrahydro-4H-pyrrolo[3,2-c]pyridin-4-one;
(7R,7S)-7-isopropyl-2-pyridin-4-yl-1,5,6,7-tetrahydro-4H-pyrrolo[3,2-c]pyridin-4-one;
(7R,7S)-7-sec-butyl-2-pyridin-4-yl-1,5,6,7-tetrahydro-4H-pyrrolo[3,2-c]pyridin-4-one;
2'-pyridin-4-yl-5',6'-dihydrospiro[cyclopropane-1,7'-pyrrolo[3,2-c]pyridin]-4'(1'H)-one;
(7R,7S)-7-isobutyl-2-pyridin-4-yl-1,5,6,7-tetrahydro-4H-pyrrolo[3,2-c]pyridin-4-one hydrochloride;
(7R,7S)-7-ethyl-2-pyridin-4-yl-1,5,6,7-tetrahydro-4H-pyrrolo[3,2-c]pyridin-4-one hydrochloride;
7,7-dimethyl-2-pyridin-4-yl-1,5,6,7-tetrahydro-4H-pyrrolo[3,2-c]pyridin-4-one hydrochloride;
7,7-diethyl-2-pyridin-4-yl-1,5,6,7-tetrahydro-4H-pyrrolo[3,2-c]pyridin-4-one hydrochloride;
(7R,7S)-2-(3-fluoropyridin-4-yl)-7-isopropyl-1,5,6,7-tetrahydro-4H-pyrrolo[3,2-c]pyridin-4-one;
(7R,7S)-2-(3-fluoropyridin-4-yl)-7-isopropyl-1,5,6,7-tetrahydro-4H-pyrrolo[3,2-c]pyridin-4-one;
(7R,7S)-2-(3-fluoropyridin-4-yl)-7-ethyl-1,5,6,7-tetrahydro-4H-pyrrolo[3,2-c]pyridin-4-one and
2-[2-(cyclopentylamino)pyridin-4-yl]-1,5,6,7-tetrahydro-4H-pyrrolo[3.2-c]pyridin-4-one.

**10.** A pharmaceutical composition comprising a therapeutically effective amount of a compound of formula (I) or a pharmaceutically acceptable salt thereof, as defined in claim 1, and at least one pharmaceutically acceptable excipient, carrier and/or diluent.

**11.** A pharmaceutical composition according to claim 10 further comprising one or more chemotherapeutic agents.

**12.** A product or kit comprising a compound of formula (I) or a pharmaceutically acceptable salt thereof, as defined in claim 1, or pharmaceutical compositions thereof as defined in claim 10, and one or more chemotherapeutic agent, as a combined preparation for simultaneous, separate or sequential use in anticancer therapy.

**13.** A compound of formula (I) or a pharmaceutically acceptable salt thereof, as defined in claim 1, for use as a medicament.

**14.** Use of a compound of formula (I) or a pharmaceutically acceptable salt thereof, as defined in claim 1, in the manufacture of a medicament with antitumor activity.

## Patentansprüche

**1.** Verwendung einer Verbindung von Formel (I) für die Herstellung eines Medikaments zur Behandlung zellproliferativer Störungen, verursacht durch und/oder assoziiert mit einer veränderten Proteinkinaseaktivität, gewählt aus der Gruppe bestehend aus Krebs, Alzheimer-Krankheit, Virusinfektionen, Autoimmunerkrankungen, neurodegenerativen Störungen, benigner Prostatahyperplasie, familiärer Adenomatosis Polyposis, Neurofibromatose, glatter Gefäßzell-Proliferation assoziiert mit Atherosklerose, Lungenfibrose, Arthritis, Arthritis Glomerulonephritis und postoperativer Stenose und Restenose, worin die Verbindung in einer wirksamen Menge an ein Säugetier, das dies benötigt, verabreicht werden soll,

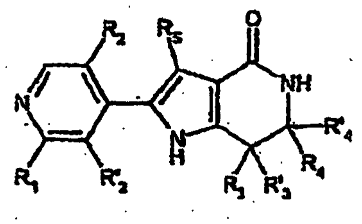

(I)

worin
$R_1$ ein Wasserstoffatom, Amino oder $C_3$-$C_7$ Cycloalkylamino ist;
$R_2$ und $R'_2$ sind, jeweils unabhängig, ein Wasserstoff- oder Halogenatom oder eine gerade oder verzweigte $C_1$-$C_6$ Alkylgruppe; oder, zusammengenommen mit der Pyridinbindung, an welche sie gebunden sind, können $R_1$ und $R'_2$

eine zweiwertige -NH-CH=CH-Gruppe bilden;
$R_3$, $R'_3$, $R_4$ und $R'_4$ sind, jeweils unabhängig, ein Wasserstoffatom oder eine Gruppe gewählt aus geradem oder verzweigtem $C_1$-$C_6$ Alkyl, $C_3$-$C_6$ Cycloalkyl, Heterocyclyl, Aryl, Cycloalkyl-Alkyl, Heterocyclyl-Alkyl oder Aryl-Alkyl; oder $R_3$ und $R'_3$ oder $R_4$ und $R'_4$ bilden zusammengenommen eine $C_3$-$C_6$ Cycloalkylgruppe;
$R_5$ ist ein Wasserstoff- oder Halogenatom oder es ist eine gerade oder verzweigte $C_1$-$C_6$ Alkylgruppe, und pharmazeutisch verträgliche Salze davon.

2. Die Verwendung gemäß Anspruch 1, worin der Krebs gewählt ist aus der Gruppe bestehend aus Karzinom, Plattenepithelkarzinom, hämatopoietischen Tumoren myeloider oder lymphoider Abstammung, Tumoren mesenchymalen Ursprungs, Tumoren des zentralen und peripheren Nervensystems, Melanom, Seminom, Teratokarzinom, Osteosarkom, Xeroderma Pigmentosum, Keratoakanthom, Schilddrüsenfollikelkrebs und Kaposi-Sarkom.

3. Die Verwendung gemäß Anspruch 1, worin das Säugetier, das dies benötigt, weiter einer Bestrahlungstherapie oder einem Chemotherapieregime in Kombination mit mindestens einem cytostatischen oder cytotoxischen Wirkstoff unterworfen werden soll.

4. Die Verwendung gemäß Anspruch 1, worin das Säugetier, das dies benötigt, ein Mensch ist.

5. Eine Verwendung von Formel (I)

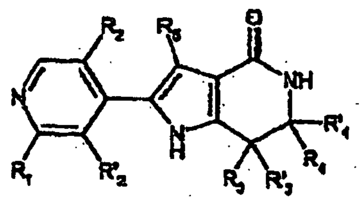

(I)

worin
$R_1$ ein Wasserstoffatom, Amino oder $C_3$-$C_7$ Cycloalkylamino ist;
$R_2$ und $R'_2$ sind, jeweils unabhängig, ein Wasserstoff- oder Halogenatom oder eine gerade oder verzweigte $C_1$-$C_6$ Alkylgruppe; oder, zusammengenommen mit der Pyridinbindung, an welche sie gebunden sind, können $R_1$ und $R'_2$ eine divalente -NH-CH=CH-Gruppe bilden;
$R_3$, $R'_3$, $R_4$ und $R'_4$ sind, jeweils unabhängig, ein Wasserstoffatom oder eine Gruppe gewählt aus geradem oder verzweigtem $C_1$-$C_6$ Alkyl, $C_3$-$C_6$ Cycloalkyl, Heterocyclyl, Aryl, Cycloalkyl-Alkyl, Heterocyclyl-Alkyl oder Aryl-Alkyl; oder $R_3$ und $R'_3$ oder $R_4$ und $R'_4$ bilden zusammengenommen eine $C_3$-$C_6$ Cycloalkylgruppe;
$R_5$ ist ein Wasserstoff- oder Halogenatom oder es ist eine gerade oder verzweigte $C_1$-$C_6$ Alkylgruppe, und pharmazeutisch verträgliche Salze davon;
vorausgesetzt, daß die Verbindung nicht 2-(2-Aminopyridin-4-yl)-1,5,6,7-tetrahydro-4H-pyrrolo[3,2-c]pyridin-4-on ist.

6. Eine Verbindung von Formel (I) wie in Anspruch 5 definiert,
worin $R_3$ und $R'_3$ beide Wasserstoffatome sind, oder eines davon ist eine Phenylgruppe und das verbleibende ist ein Wasserstoffatom; und $R_1$, $R_2$, $R'_2$, $R_4$, $R'_4$ und $R_5$ sind wie in Anspruch 5 definiert.

7. Eine Verbindung von Formel (I) wie in Anspruch 5 definiert,
worin $R_4$ und $R'_4$ beide Wasserstoffatome oder Methylgruppen sind, oder eines davon ist eine Methyl- oder Phenylgruppe und das verbleibende ist ein Wasserstoffatom; und $R_1$, $R_2$, $R'_2$, $R_3$, $R'_3$ und $R_5$ sind wie in Anspruch 5 definiert.

8. Eine Verbindung von Formel (I) wie in Anspruch 5 definiert,
worin $R_1$ Wasserstoff ist und worin $R_2$ und $R'_2$ jeweils unabhängig Wasserstoff oder Halogenatome sind; $R_6$ ist ein Wasserstoffatom oder eine Methylgruppe und $R_3$, $R'_3$, $R_4$ und $R'_4$ sind wie oben definiert.

**9.** Eine Verbindung von Formel (I) gemäß Anspruch 5, wahlweise in der Form eines pharmazeutisch verträglichen Salzes davon, gewählt aus der Gruppe bestehend aus:

2-(1H-Pyrrolo[2,3-b]pyridin-4-yl)-1,5,6,7-tetrahydro-4H-pyrrolo[3,2-c]pyridin-4-on Hydrochlorid;
2-Pyridin-4-yl-1,5,6,7-tetrahydro-4H-pyrrolo[3,2-c]pyridin-4-on Hydrochlorid;
2-(3-Fluorpyridin-4-yl)-1,5,6,7-tetrahydro-4H-pyrrolo[3,2-c]pyridin-4-on Hydrochlorid;
3-Methyl-2-pyridin-4-yl-1,5,6,7-tetrahydro-4H-pyrrolo[3,2-c]pyridin-4-on Hydrochlorid;
2-(1H-Pyrrolo[2,3-b]pyridin-4-yl)-1,5,6,7-tetrahydro-4H-pyrrolo[3,2-c]pyridin-4-on;
(6S)-6-Methyl-2-pyridin-4-yl-1,5,6,7-tetrahydro-4H-pyrrolo[3,2-c]pyridin-4-on;
(6R,6S)-6-Benzyl-2-pyridin-4-yl-1,5,6,7-tetrahydro-4H-pyrrolo[3,2-c]pyridin-4-on;
(6R oder 6S)-6-Benzyl-2-pyridin-4-yl-1,5,6,7-tetrahydro-4H-pyrrolo[3,2-c]pyridin-4-on;
(6R,6S)-6-(2-Phenylethyl)-2-pyridin-4-yl-1,5,6,7-tetrahydro-4H-pyrrolo[3,2-c]pyridin-4-on Hydrochlorid;
(7R,7S)-7-Methyl-2-pyridin-4-yl-1,5,6,7-tetrahydro-4H-pyrrolo[3,2-c]pyridin-4-on Hydrochlorid;
(7R oder 7S)-7-Methyl-2-pyridin-1,5,6,7-tetrahydro-4H-pyrrolo[3,2-c]pyridin-4-on;
(6R,6S)-6-Isopropyl-2-pyridin-4-yl-1,5,6,7-tetrahydro-4H-pyrrolo[3,2-c]pyridin-4-on Hydrochlorid;
(7R,7S)-7-Benzyl-2-pyridin-4-yl-1,5,6,7-tetrahydro-4H-pyrrolo[3,2-c]pyridin-4-on;
(6R,6S)-6-Cyclopropyl-2-pyridin-4-yl-1,5,6,7-tetrahydro-4H-pyrrolo[3,2-c]pyridin-4-on;
(6R,6S)-6-Cyclohexyl-2-pyridin-4-yl-1,5,6,7-tetrahydro-4H-pyrrolo[3,2-c]pyridin-4-on;
(7R,7S)-7-Isopropyl-2-pyridin-4-yl-1,5,6,7-tetrahydro-4H-pyrrolo[3,2-c]pyridin-4-on;
(7R,75)-7-sec-Butyl-2-pyridin-4-yl-1,5,6,7-tetrahydro-4H-pyrrolo[3,2-c]pyridin-4-on;
2'-Pyridin-4-yl-5',6'-dihydrospiro[cyclopropan-1,7'-pyrrolo[3,2-c]pyridin]-4'(1'H)-on;
(7R,7S)-7-Isobutyl-2-pyridin-4-yl-1,5,6,7-tetrahydro-4H-pyrrolo[3,2-c]pyridin-4-on Hydrochlorid;
(7R,7S)-7-Ethyl-2-pyridin-4-yl-1,5,6,7-tetrahydro-4H-pyrrolo[3,2-c]pyridin-4-on Hydrochlorid;
7,7-Dimethyl-2-pyridin-4-yl-1,5,6,7-tetrahydro-4H-pyrrolo[3,2-c]pyridin-4-on Hydrochlorid;
7,7-Diethyl-2-pyridin-4-yl-1,5,6,7-tetrahydro-4H-pyrrolo[3,2-c]pyridin-4-on Hydrochlorid;
(7R,7S)-2-(3-Fluorpyridin-4-yl)-7-isopropyl-1,5,6,7-tetrahydro-4H-pyrrolo[3,2-c]pyridin-4-on;
(7R,7S)-2-(3-Fluorpyridin-4-yl)-7-isopropyl-1,5,6,7-tetrahydro-4H-pyrrolo[3,2-c]pyridin-4-on;
(7R,7S)-2-(3-Fluorpyridin-4-yl)-7-ethyl-1,5,6,7-tetrahydro-4H-pyrrolo[3,2-c]pyridin-4-on und
2-[2-(Cyclopentylamino)pyridin-4-yl]-1,5,6,7-tetrahydro-4H-pyrrolo[3,2-c]pyridin-4-on.

**10.** Eine pharmazeutische Zusammensetzung, die eine therapeutisch wirksame Menge einer Verbindung von Formel (I) oder ein pharmazeutisch verträgliches Salz davon, wie in Anspruch 1 definiert, und mindestens ein pharmazeutisch verträgliches Bindemittel, Träger und/oder Verdünnungsmittel umfasst.

**11.** Eine pharmazeutische Zusammensetzung gemäß Anspruch 10, die weiter einen oder mehrere chemotherapeutische Wirkstoffe umfaßt.

**12.** Ein Produkt oder Kit, umfassend eine Verbindung von Formel (I) oder ein pharmazeutisch verträgliches Salz davon, wie in Anspruch 1 definiert, oder pharmazeutische Zusammensetzungen davon wie in Anspruch 10 definiert, und einen oder mehrere chemotherapeutische Wirkstoffe, als eine kombinierte Zubereitung für die gleichzeitige, getrennte oder aufeinanderfolgende Verwendung in der Antikrebstherapie.

**13.** Eine Verbindung von Formel (I) oder ein pharmazeutisch verträgliches Salz davon, wie in Anspruch 1 definiert, für die Verwendung als ein Medikament.

**14.** Verwendung einer Verbindung von Formel (I) oder ein pharmazeutisch verträgliches Salz davon, wie in Anspruch 1 definiert, in der Herstellung eines Medikaments mit Antitumoraktivität.

## Revendications

**1.** Utilisation d'un composé de formule (I) pour la production d'un médicament destiné au traitement de troubles de la prolifération cellulaire provoqués par et/ou associés à une activité de protéine-kinase modifiée, choisis dans le groupe consistant en le cancer, la maladie d'Alzheimer, des infections virales, des maladies auto-immunes, des troubles neurodégénératifs, l'hyperplasie prostatique bénigne, l'adénomatose polypeuse familiale, la neurofibromatose, le psoriasis, la prolifération de cellules du tissu lisse vasculaire associée à l'athérosclérose, la fibrose pulmonaire, l'arthrite, la glomérulonéphrite et la sténose et la resténose postchirurgicales, dans laquelle le composé est destiné à être administré en une quantité efficace à un mammifère en ayant besoin

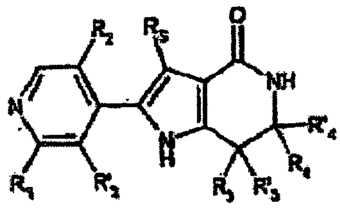 (I)

formule dans laquelle
$R_1$ représente un atome d'hydrogène ou un groupe cycloalkylamino en $C_3$ à $C_7$ ;
$R_2$ et $R'_2$ représentent, chacun indépendamment, un atome d'hydrogène ou d'halogène ou un groupe alkyle en $C_1$ à $C_6$ droit ou ramifié ou bien, pris conjointement avec la liaison pyridine à laquelle ils sont liés, $R_1$ et $R'_2$ peuvent former un groupe -NH-CH=CH- divalent ;
$R_3$, $R'_3$, $R_4$ et $R'_4$ représentent, chacun indépendamment, un atome d'hydrogène ou un groupe choisi entre des groupes alkyle en $C_1$ à $C_6$ droits ou ramifiés, cycloalkyle en $C_3$ à $C_6$, hétérocyclyle, aryle, cycloalkylalkyle, hétérocyclylalkyle et arylalkyle, ou bien $R_3$ et $R'_3$ ou $R_4$ et $R'_4$, pris conjointement, forment un groupe cycloalkyle en $C_3$ à $C_6$ ;
$R_5$ représente un atome d'hydrogène ou d'halogène ou représente un groupe alkyle en $C_1$ à $C_6$ droit ou ramifié, et de ses sels pharmaceutiquement acceptables.

2. Utilisation suivant la revendication 1, dans laquelle le cancer est choisi dans le groupe consistant en carcinome, un carcinome à cellules squameuses, des tumeurs hématopoïétiques de la lignée myéloïde ou lymphoïde, des tumeurs d'origine mésenchymateuse, des tumeurs du système nerveux central et du système nerveux périphérique, un mélanome, un séminome, un tératocarcinome, un ostéosarcome, le xeroderma pigmentosum, un kératoxanthome, le cancer folliculaire de la thyroïde et le sarcome de Kaposi.

3. Utilisation suivant la revendication 1, dans laquelle le mammifère en ayant besoin est destiné en outre à être soumis à un schéma de radiothérapie ou chimiothérapie en association avec au moins un agent cytostatique ou cytotoxique.

4. Utilisation suivant la revendication 1, dans laquelle le mammifère en ayant besoin est un être humain.

5. Composé de formule (I)

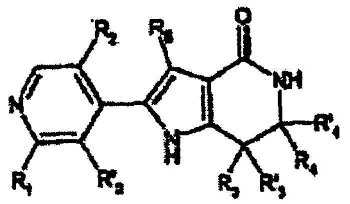 (I)

dans laquelle
$R_1$ représente un atome d'hydrogène ou un groupe cycloalkylamino en $C_3$ à $C_7$ ;
$R_2$ et $R'_2$ représentent, chacun indépendamment, un atome d'hydrogène ou d'halogène ou un groupe alkyle en $C_1$ à $C_6$ droit ou ramifié ; ou bien, pris conjointement avec la liaison pyridine à laquelle ils sont liés, $R_1$ et $R'_2$ peuvent former un groupe -NH-CH=CH- divalent ;
$R_3$, $R'_3$, $R_4$ et $R'_4$ représentent, chacun indépendamment, un atome d'hydrogène ou un groupe choisi entre des groupes alkyle en $C_1$ à $C_6$ droits ou ramifiés, cycloalkyle en $C_3$ à $C_8$, hétérocyclyle, aryle, cycloalkylalkyle, hétérocyclylalkyle et arylalkyle, ou bien $R_3$ et $R'_3$ ou $R_4$ et $R'_4$, pris conjointement, forment un groupe cycloalkyle en $C_3$ à $C_6$ ;
$R_5$ représente un atome d'hydrogène ou d'halogène ou représente un groupe alkyle en $C_1$ à $C_6$ droit ou ramifié, et ses sels pharmaceutiquement acceptables ;
sous réserve que le composé ne soit pas la 2-(2-amino-pyridine-4-yl)-1,5,8,7-tétrahydro-4H-pyrrolo[3,2-c]pyridine-4-one.

6. Composé de formule (I) suivant la revendication 5, dans lequel $R_3$ et $R'_3$ représentent l'un et l'autre un atome d'hydrogène ou l'un d'entre eux représente un groupe phényle et le groupe restant représente un atome d'hydrogène et $R_1$, $R_2$, $R'_2$, $R_4$, $R'_4$ et $R_5$ sont tels que définis dans la revendication 5.

7. Composé de formule (I) suivant la revendication 5, dans lequel $R_4$ et $R'_4$ représentent l'un et l'autre un atome d'hydrogène ou un groupe méthyle ou bien l'un d'entre eux représente un groupe méthyle ou un groupe phényle et le groupe restant représente un atome d'hydrogène ; et $R_1$, $R_2$, $R'_2$, $R_3$, $R'_3$ et $R_5$ sont tels que définis dans la revendication 5.

8. Composé de formule (I) suivant la revendication 5, dans lequel $R_1$ représente un atome d'hydrogène et dans lequel $R_2$ et $R'_2$ représentent, chacun indépendamment, un atome d'hydrogène ou d'halogène, $R_5$ représente un atome d'hydrogène ou un groupe méthyle et $R_3$, $R'_3$, $R_4$ et $R'_4$ répondent aux définitions précitées.

9. Composé de formule (I) suivant la revendication 5, facultativement sous forme d'un de ses sels pharmaceutiquement acceptables, choisi dans le groupe consistant en :

le chlorhydrate de 2-(1H-pyrrolo[2,3-b]pyridine-4-yl)-1,5,6,7-tétrahydro-4H-pyrrolo[3,2-c]pyridine-4-one ;
le chlorhydrate de 2-pyridine-4-yl-1,5,6,7-tétrahydro-4H-pyrrolo[3,2-c]pyridine-4-one ;
le chlorhydrate de 2-(3-fluoropyridine-4-yl)-1,5,6,7-tétrahydro-4H-pyrrolo[3,2-c]pyridine-4-one ;
le chlorhydrate de 3-méthyl-2-pyridine-4-yl-1,5,6,7-tétrahydro-4H-pyrrolo[3,2-c]pyridine-4-one ;
la 2-(1H-pyrrolo[2,3-b]pyridine-4-yl)-1,5,6,7-tétrahydro-4H-pyrrolo[3,2-c]pyridine-4-one ;
la (6S)-6-méthyl-2-pyridine-4-yl-1,5,6,7-tétrahydro-4H-pyrrolo[3,2-c]pyridine-4-one ;
la (6R,6S)-6-benzyl-2-pyridine-4-yl-1,5,6,7-tétrahydro-4H-pyrrolo[3,2-c]pyridine-4-one ;
la (6R ou 6S)-6-benzyl-2-pyridine-4-yl-1,5,6,7-tétrahydro-4H-pyrrolo[3,2-c]pyridine-4-one ;
le chlorhydrate de (6R,6S)-6-(2-phényléthyl)-2-pyridine-4-yl-1,5,6,7-tétrahydro-4H-pyrrolo[3,2-c]pyridine-4-one ;
le chlorhydrate de (7R,7S)-7-méthyl-2-pyridine-4-yl-1,5,6,7-tétrahydro-4H-pyrrolo[3,2-c]pyridine-4-one ;
la (7R ou 7S)-7-méthyl-2-pyridine-4-yl-1,5,6,7-tétrahydro- . 4H-pyrrolo[3,2-c]pyridine-4-one ;
le chlorhydrate de (6R,6S)-6-isopropyl-2-pyridine-4-yl-1,5,6,7-tétrahydro-4H-pyrrolo[3,2-c]pyridine-4-one ;
la (7R,7S)-7-benzyl-2-pyridine-4-yl-1,5,6,7-tétrahydro-4H-pyrrolo[3,2-c]pyridine-4-one ;
la (6R,6S)-6-cyclopropyl-2-pyridine-4-yl-1,5,6,7-tétrahydro-4H-pyrrolo[3,2-c]pyridine-4-one ;
la (6R,6S)-6-cyclohexyl-2-pyridine-4-yl-1,5,6,7-tétrahydro-4H-pyrrolo[3,2-c]pyridine-4-one ;
la (7R,7S)-7-isopropyl-2-pyridine-4-yl-1,5,6,7-tétrahydro-4H-pyrrolo[3,2-c]pyridine-4-one ;
la (7R,7S)-7-sec.-butyl-2-pyridine-4-yl-1,5,6,7-tétrahydro-4H-pyrrolo[3,2-c]pyridine-4-one ;
la 2'-pyridine-4-yl-5',6'-dihydrospiro[cyclopropane-1,7'-pyrrolo[3,2-c]pyridine]-4'(1'H)-one ;
le chlorhydrate de (7R,7S)-7-isopropyl-2-pyridine-4-yl-1,5,6,7-tétrahydro-4H-pyrrolo[3,2-c]pyridine-4-one ;
le chlorhydrate de (7R,7S)-7-éthyl-2-pyridine-4-yl-1,5,6,7-tétrahydro-4H-pyrrolo[3,2-c]pyridine-4-one ;
le chlorhydrate de 7,7-diméthyl-2-pyridine-4-yl-1,5,6,7-tétrahydro-4H-pyrrolo[3,2-c]pyridine-4-one ;
le chlorhydrate de 7,7-diéthyl-2-pyridine-4-yl-1,5,6,7-tétrahydro-4H-pyrrolo[3,2-c]pyridine-4-one ;
la (7R,7S)-2-(3-fluoropyridine-4-yl)-7-isopropyl-1,5,6,7-tétrahydro-4H-pyrrolo[3,2-c]pyridine-4-one ;
la (7R,7S)-2-(3-fluoropyridine-4-yl)-7-isobutyl-1,5,6,7-tétrahydro-4H-pyrrolo[3,2-c]pyridine-4-one ;
la (7R,7S)-2-(3-fluoropyridine-4-yl)-7-éthyl-1,5,6,7-tétrahydro-4H-pyrrolo[3,2-c]pyridine-4-one ; et
la 2-[2-(cyclopentylamino)pyridine-4-yl]-1,5,6,7-tétrahydro-4H-pyrrolo[3,2-c]pyridine-4-one.

10. Composition pharmaceutique comprenant une quantité thérapeutiquement efficace d'un composé de formule (I) ou d'un de ses sels pharmaceutiquement acceptables, tel que défini dans la revendication 1, et au moins un excipient, support et/ou diluant pharmaceutiquement acceptable.

11. Composition pharmaceutique suivant la revendication 10, comprenant en outre un ou plusieurs agents chimiothérapeutiques.

12. Produit ou kit comprenant un composé de formule (I) ou un de se sels pharmaceutiquement acceptables, tel que défini dans la revendication 1, ou compositions pharmaceutiques le contenant telles que définies dans la revendication 10, et un ou plusieurs agents chimiothérapeutiques, sous forme d'une préparation combinée pour une utilisation simultanée, séparée ou séquentielle en thérapie anticancéreuse.

13. Composé de formule (I) ou un de ses sels pharmaceutiquement acceptables, tel que défini dans la revendication 1, destiné à être utilisé comme médicament.

14. Utilisation d'un composé de formule (I) ou d'un de ses sels pharmaceutiquement acceptables, telle que définie dans la revendication 1, dans la production d'un médicament ayant une activité antitumorale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03027114 A
- WO 0212242 A
- WO 0069846 A
- WO 0198299 A
- WO 03014090 A
- WO 03028720 A
- WO 04058762 A
- US 60434962 B


### Non-patent literature cited in the description


- *Current Opinion in Chemical Biology,* 1999, vol. 3, 459-465
- **MONTAGNOLL A.** *EMBO Journal,* 2002, vol. 21 (12), 3171-3181